(19) Europäisches Patentamt / European Patent Office / Office européen des brevets

(11) **EP 1 871 265 B1**

(12) **FASCICULE DE BREVET EUROPEEN**

(45) Date de publication et mention
de la délivrance du brevet:
**03.10.2012 Bulletin 2012/40**

(21) Numéro de dépôt: **06709387.2**

(22) Date de dépôt: **28.02.2006**

(51) Int Cl.:
*A61B 17/34* *(2006.01)*     *A61B 19/00* *(2006.01)*

(86) Numéro de dépôt international:
**PCT/FR2006/000451**

(87) Numéro de publication internationale:
**WO 2006/092496 (08.09.2006 Gazette 2006/36)**

(54) **DISPOSITIF DE MAINTIEN ET DE DEPLACEMENT CONTROLE EN TRANSLATION D'UN CORPS ALLONGE**

VORRICHTUNG ZUR UNTERSTÜTZUNG EINES LÄNGLICHEN KÖRPERS UND FÜR DIE KONTROLLIERTE TRANSLATORISCHE BEWEGUNG DESSELBEN

DEVICE FOR SUPPORTING AN ELONGATED BODY AND FOR THE CONTROLLED TRANSLATIONAL MOVEMENT OF SAME

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **28.02.2005 FR 0502016**

(43) Date de publication de la demande:
**02.01.2008 Bulletin 2008/01**

(73) Titulaires:
• **Institut National Des Sciences Appliquees
67000 Strasbourg (FR)**
• **Institut de Recherche sur les Cancers de l'Appareil Digestif IRCAD
67000 Strasbourg (FR)**
• **Université de Strasbourg
67000 Strasbourg (FR)**
• **Centre National de la Recherche Scientifique
75016 Paris (FR)**

(72) Inventeurs:
• **DE MATHELIN, Michel
F-67000 Strasbourg (FR)**
• **MAURIN, Benjamin
74960 Cran Gevrier (FR)**
• **BAYLE, Bernard
F-67000 Strsabourg (FR)**
• **GANGLOFF, Jacques
F-67350 Mulhausen (FR)**
• **PICCIN, Olivier
F-67170 Mittelhausen (FR)**

(74) Mandataire: **Nuss, Pierre et al
Cabinet Nuss
10 Rue Jacques Kablé
67080 Strasbourg Cedex (FR)**

(56) Documents cités:
WO-A-00/28882          US-A- 4 765 669
US-A1- 2003 171 670    US-B1- 6 726 675

EP 1 871 265 B1

## Description

La présente invention concerne le domaine de la robotique, en particulier en relation avec le déplacement contrôlé d'un objet, corps, pièce ou analogue, et a pour objet un dispositif de maintien et de déplacement contrôlé d'un corps allongé en forme de tige ou analogue.

Il existe de nombreuses applications dans lesquelles un objet, notamment allongé, doit être déplacé de manière précise et contrôlée par un dispositif mécanique, électro-mécanique, mécano-pneumatique ou autre du type machine, appareil ou instrument, ce de manière semi-automatique, sans intervention directe d'un utilisateur ou opérateur, quoiqu'éventuellement sous le contrôle de ce dernier.

On connaît en particulier des applications dans lesquelles un corps allongé doit être déplacé en translation dans sa direction longitudinale, par exemple en vue de son enfoncement dans un autre corps, en imitant éventuellement un mouvement traditionnellement réalisé par un opérateur humain.

Tel est notamment le cas pour les actes médicaux assistés par ordinateur, par exemple dans les procédures percutanées.

De telles procédures opératoires sont souvent réalisées sous imagerie médicale, en particulier sous scannographique X, permettant ainsi au praticien de contrôler en temps réel son intervention.

Toutefois, durant ces procédures le praticien est exposé à des rayonnements nocifs de manière répétitive, notamment à des rayons X dont les intensités sont encore accrues en scannographie fluoroscopique.

Or, les procédures médicales percutanées sous imagerie scanner sont des actes médicaux nécessitant une grande précision et une protection simultanée du patient et du chirurgien, ces procédures consistant à insérer une aiguille dans un organe cible à traiter ou à sonder (prélèvement de tissus, injection localisée, destruction de tumeur).

Il existe donc actuellement un besoin et une demande pour un système d'insertion contrôlée d'aiguilles.

Un tel système doit être à la fois précis et compact, du fait du faible volume disponible dans le scannographe ou analogue, entre la poitrine ou l'abdomen du patient et la paroi intérieure du tunnel de l'appareil.

La présente invention concerne plus particulièrement, mais non limitativement, un dispositif d'insertion et d'extraction d'aiguille destiné à être monté sur un support réglable en position et en orientation d'un dispositif robotique de positionnement et d'orientation, compatible avec l'application, garantissant la bonne incidence de l'aiguille et autorisant une éventuelle rotation de cette dernière autour de son axe.

On connaît déjà un dispositif robotisé d'insertion d'aiguilles à actionnement pneumatique dans lequel l'aiguille est pincée par une mâchoire mobile effectuant un déplacement en translation. Toutefois, la course et la force de pression maximale disponible sont limitées, et ne permettent pas la manipulation d'aiguille de grande longueur.

On connaît aussi des dispositifs robotisés compacts d'insertion d'aiguilles basés sur un entraînement par friction, par exemple par l'intermédiaire de galets ou de rouleaux entraînés en rotation et recevant l'aiguille entre eux. Du fait de leur conception, ces dispositifs sont compacts, permettent une grande course de déplacement et fournissent naturellement une limitation de l'effort d'insertion. Toutefois, cette limitation d'efforts d'insertion n'est pas précise, ni reproductible (elle dépend de nombreux facteurs : états de surface des galets d'entraînement et de l'aiguille, grosseur de l'aiguille, etc...). De plus, un tel entraînement par friction ne permet pas de définir la position précise de la pointe de l'aiguille (en particulier durant l'insertion), ni de fournir directement la course effective et instantanée de l'aiguille, ni d'évaluer les efforts appliqués à cette dernière, ni d'assurer un maintien en position sûre de l'aiguille à l'état inséré (ou dans une position d'arrêt en cours d'insertion).

Enfin, d'autres dispositifs d'enfoncement contrôlé d'une aiguille ou analogue sont également connus lesquels ne permettent par contre pas une rotation de l'aiguille autour de son axe, ni un éventuel relâchement rapide de l'aiguille avec un maintien lâche ou avec une liberté relative, associé à une reprise garantie de ladite aiguille pour un nouveau serrage. De plus, certains au moins de ces dispositifs nécessitent des aiguilles de forme particulière. US 6726675 décrit un système d'insertion pour un cathétère.

La présente invention a pour but de pallier au moins certains de ces inconvénients précités, en particulier, mais non limitativement, dans le contexte des procédures chirurgicales percutanées.

A cet effet, la présente invention a pour objet un dispositif de maintien et de déplacement contrôlé en translation comme défini dans la revendication 1, dans sa direction longitudinale, d'un corps allongé en forme de tige, notamment d'une aiguille, et en particulier d'une aiguille pour procédure médicale percutanée, dispositif destiné à être monté sur un support réglable de manière contrôlée en position et en orientation, tel que par exemple une plateforme ou un bras d'un dispositif robotique de positionnement et d'orientation, ce dispositif comprenant essentiellement, d'une part, un premier organe de préhension du corps allongé, déplaçable par rapport au support de manière commandée et/ou contrôlée dans la direction de translation avec une course maximale prédéterminée, et, d'autre part, un second organe fixe par rapport au support et apte à guider le corps allongé lorsque ce dernier est déplacé par le premier organe mobile de préhension et, le cas échéant, à maintenir ledit corps allongé en position lorsque ce dernier n'est pas en prise avec ledit premier organe, les deux organes étant alignés dans la direction de translation et le premier organe pouvant être déplacé de manière alternative entre une position proximale du second organe et une position distante de ce dernier, dispositif, caractérisé en ce que l'organe de préhension mobile comprend des moyens de serrage par pincement et avec blocage du corps allongé et consiste en un mandrin recevant le corps allongé de manière traversante, ledit mandrin pouvant être déplacé en rotation avec le corps allongé autour de l'axe de translation.

L'invention sera mieux comprise, grâce à la description ci-après, qui se rapporte à des modes de réalisation préférés, donnés à titre d'exemples non limitatifs, et expliqués avec référence aux dessins schématiques annexés, dans lesquels :

les figures 1(a) à 1(e) sont des représentations schématiques illustrant les différentes phases opératoires d'un cycle de déplacement d'un corps allongé réalisé par l'intermédiaire des deux organes d'un dispositif de maintien et de déplacement contrôlé selon un premier mode de réalisation de l'invention ;

la figure 2 est une vue en perspective d'un mode de réalisation des deux organes de la figure 1 et de leurs moyens de commande et de support faisant partie du dispositif selon un premier mode de réalisation de l'invention et formant un module de ce dernier ;

la figure 3 est une vue éclatée en perspective d'un premier mode de réalisation du dispositif selon l'invention, montrant ses différents modules constitutifs, ainsi qu'une plateforme de montage correspondant d'un dispositif robotique pour son fonctionnement et son orientation ;

les figures 4A et 4B sont des représentations schématiques, respectivement de dessus et en élévation latérale, montrant la disposition des capteurs de forces ou d'efforts faisant partie du dispositif selon l'invention ;

les figures 5A et 5B sont des vues en perspective de dessus du module regroupant les mécanismes d'actionnement faisant partie du dispositif de la figure 3, respectivement capoté et décapoté ;

la figure 6 est une vue de détail, en perspective du chariot portant le premier organe de préhension mobile et faisant partie du dispositif des figures 2 et 3 ;

la figure 7 est une vue de détail montrant l'actionnement du mandrin coulissant formant le premier organe de préhension, tel que représenté sur les figures 2 et 6 ;

les figures 8A et 8B sont des vues en perspective, respectivement à l'état assemblé et à l'état éclaté, d'une première variante de réalisation d'un mandrin formant les premier et second organes du dispositif des figures 2 et 3 ;

la figure 9 est une vue de détail de la face inférieure de la couronne faisant partie du mandrin représenté sur les figures 8A et 8B ;

la figure 10 est une vue en perspective d'un dispositif robotique de positionnement et d'orientation susceptible de supporter, au niveau de sa plateforme, un dispositif selon l'invention ;

les figures 11A et 11B sont des vues en perspective, respectivement à l'état assemblé et en éclaté, d'une première variante de réalisation d'un mandrin en relation avec d'autres modes de réalisation du dispositif selon l'invention ;

les figures 12A et 12B sont des vues en perspective respectivement à l'état assemblé et en éclaté, d'une seconde variante de réalisation d'un mandrin en relation avec d'autres modes de réalisation du dispositif selon l'invention ;

les figures 13A et 13B sont des vues en perspective, respectivement à l'état assemblé et en éclaté, d'un dispositif selon un deuxième mode de réalisation de l'invention, en relation avec une plateforme d'un système robotique de positionnement ;

la figure 14 est une vue en perspective d'un dispositif selon un troisième mode de réalisation de l'invention ;

la figure 15 est une vue éclatée du dispositif de la figure 14,

la figure 16 et une vue en perspective, à une échelle différente et carter enlevé, de l'ensemble motorisé de déplacement du chariot faisant partie du dispositif de la figure 15 ;

la figure 17 est une vue éclatée et à une échelle différente du chariot faisant partie du dispositif de la figure 14, et,

la figure 18 est une vue en perspective d'un dispositif robotique tel que celui de la figure 10 supportant un dispositif tel que représenté sur la figure 14.

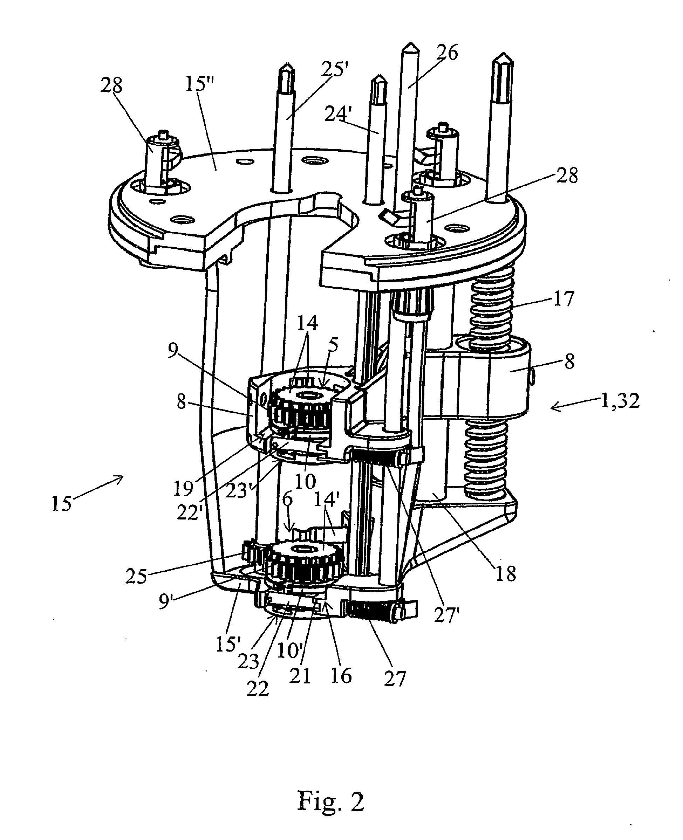

Les figures 2, 3, 13, 14 et 15 illustrent, à titre d'exemple, un dispositif 1 de maintien et de déplacement contrôlé en translation d'un corps allongé 2 en forme de tige dans sa direction longitudinale Z', notamment d'une aiguille, et en particulier une aiguille pour procédure médicale percutanée. Ce dispositif 1 notamment destiné à être monté sur un support 3 réglable, de manière contrôlée, en position et en orientation, tel que par exemple une plateforme ou un bras 3 d'un dispositif 4 robotique de positionnement et d'orientation.

Ce dispositif 1 de maintien et de déplacement contrôlé comprend essentiellement, d'une part, un premier organe 5 de préhension du corps allongé 2, déplaçable de manière commandée dans la direction de translation Z avec une course maximale prédéterminée, et, d'autre part, un second organe 6 fixe, apte à guider le corps allongé 2 lorsque ce dernier est déplacé par le premier organe mobile 5 de préhension et, le cas échéant, à maintenir ledit corps allongé 2 en position lorsque ce dernier n'est pas en prise avec ledit premier organe 5, les deux organes 5 et 6 étant alignés dans la direction de translation Z et le premier organe 5 pouvant être déplacé de manière alternative entre une position proximale du second organe 6 et une position distante de ce dernier.

Conformément à l'invention, l'organe de préhension 5 mobile comprend des moyens 7 de serrage par pincement et avec blocage du corps allongé 2, et consiste en un mandrin recevant le corps allongé 2 de manière traversante, ledit mandrin 5 pouvant être déplacé en rotation avec le corps allongé 2 autour de l'axe de translation Z.

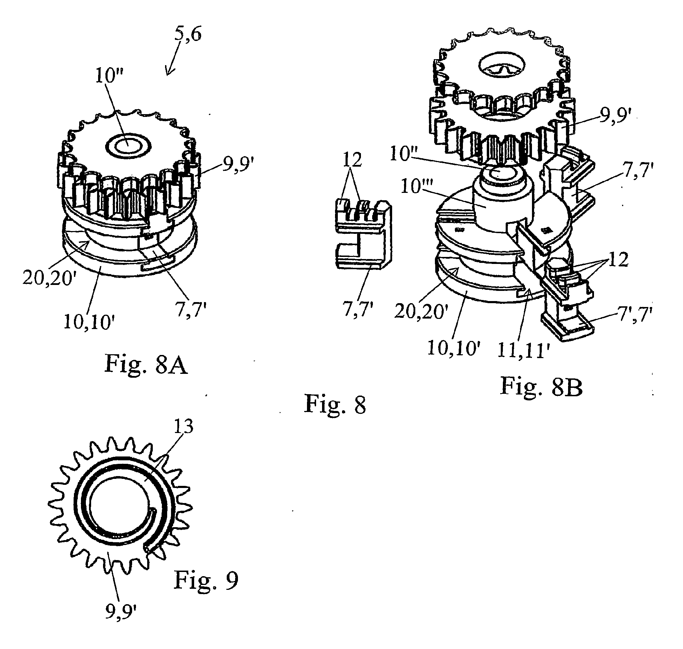

Selon l'invention et comme le montrent les figures 8, 9, 11 et 12, les mouvements de serrage et de desserrage du mandrin 5 sont commandés par un organe de manoeuvre rotatif 9, préférentiellement sous la forme d'une couronne de manoeuvre dentée ou crantée, intégrée audit mandrin 5 et en ce que les moyens de serrage 7 sous la forme de mors sont montés, avec faculté de coulissement guidé, dans un corps 10 avec un passage tubulaire 10" destiné à recevoir de manière entourante et traversante le corps allongé 2, le déplacement desdits mors 7 étant généré par un mouvement de rotation de ladite couronne de manoeuvre 9 entourant le corps 10.

Par cette forme de réalisation de l'organe de préhension 5, on peut garantir qu'après chaque relâchement du serrage du corps 2, et éventuellement associé à un déplacement en translation dudit organe, il est possible d'assurer une nouvelle prise par serrage dudit corps allongé 2, ce dernier demeurant dans le passage tubulaire 10".

Selon une première alternative de déplacement du corps allongé 2, autorisant un mouvement automatisé ou assisté de ce dernier, par exemple contrôlé par une unité informatique, il peut être prévu que le mandrin 5 formant l'organe de préhension mobile est monté sur ou dans un chariot 8 pouvant être déplacé par entraînement motorisé dans les deux sens opposés de la direction de translation Z dudit corps allongé 2, en étant guidé dans une structure support 15 allongée apte à être assemblée rigidement avec la plateforme ou le bras 3 d'un dispositif robotique 4 de positionnement et d'orientation, ladite structure support 15 étant avantageusement conformée pour autoriser un accès latéral au corps 2 engagé dans la structure support 15 et éventuellement une mise en place et une extraction en biais latéralement ou radialement du corps 2, le cas échéant avec le mandrin 5 (Figures 2, 3, 14 et 15).

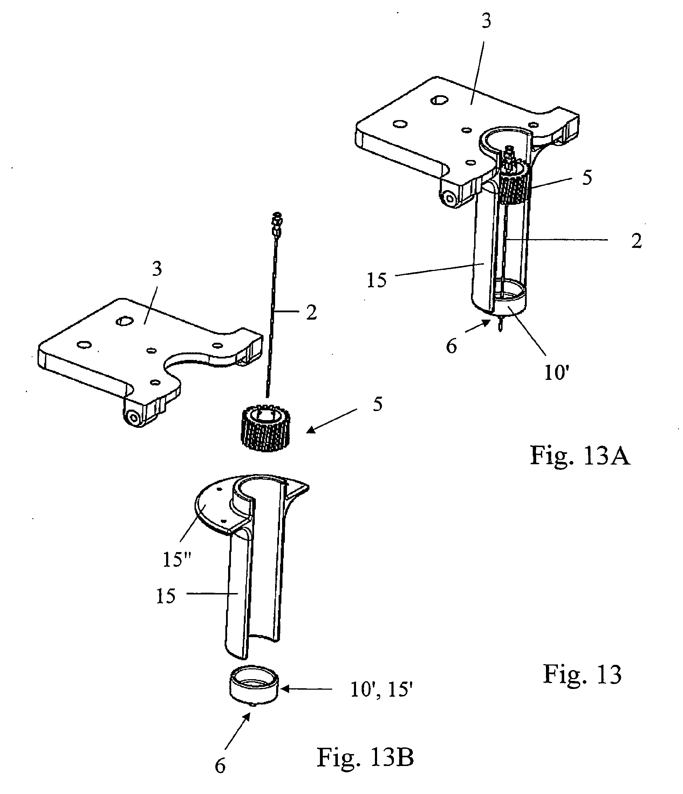

Selon une seconde alternative de déplacement du corps allongé 2, représenté aux figures 13A et 13B des dessins annexés, le mandrin 5 formant l'organe de préhension mobile est reçu directement dans un corps support 15 allongé avec faculté de coulissement guidé dans la direction de translation Z du corps allongé 2 et avec faculté de rotation autour dudit axe 2, ledit corps support 15 formant glissière pour ledit mandrin mobile 5, dont le déplacement résulte préférentiellement directement de l'action manuelle d'un opérateur, ladite structure support 15 étant avantageusement conformée pour autoriser un accès latéral au corps 2 engagé dans la structure support 15 et éventuellement une mise en place et une extraction en biais latéralement ou radialement du corps 2.

Dans ce dernier cas, l'enfoncement et la rétraction du corps 2 sont directement contrôlé par un opérateur humain, par exemple par un chirurgien lorsqu'il s'agit d'une aiguille.

Afin de permettre cet accès latéral précité, la structure support 15, qui sert de support de guidage au chariot 8 ou de guide au mandrin 5, présente avantageusement une découpe ou un dégagement latéral(e) s'étendant sensiblement sur toute la longueur de cette structure 15 dans la direction Z (voir figures précédentes).

Dans les deux alternatives de déplacement précitées, le mandrin 5 (éventuellement en coopération avec l'organe 6) sert d'adaptateur ou d'interface de montage et de guidage du corps allongé 2 dans la structure support 15, avec en plus une fonction de préhension par serrage en vue d'un entraînement en translation et en rotation dudit corps.

Comme le montrent les figures 2, 3, 14 et 15 notamment, le second organe fixe 6 est situé au niveau de l'extrémité du corps support 15 de structure allongée, qui est distale par rapport à la plateforme ou au bras 3 portant ledit dispositif 1, en étant un corps 10' rapporté sur ce corps support 15 ou formé par une partie de ce dernier.

Selon une première variante de réalisation du second organe fixe 6, ce dernier peut consister en un palier de guidage ajusté du corps allongé 2, la friction entre ce dernier et ledit palier étant au moins suffisante pour assurer un maintien dudit corps allongé 2 à l'encontre de la pesanteur.

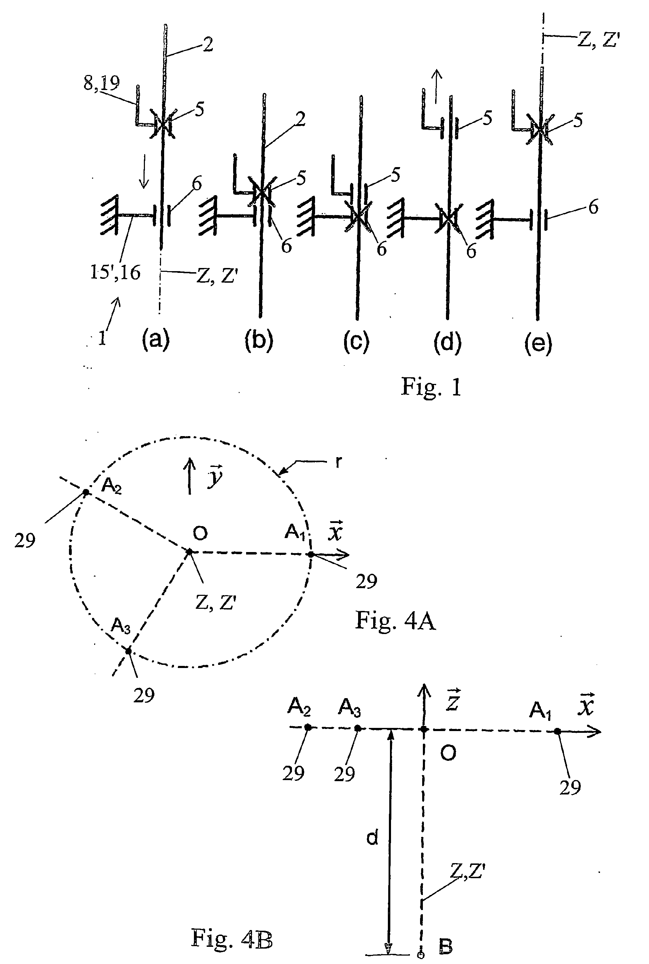

Les forces de friction seront avantageusement également suffisantes pour empêcher un déplacement axial du corps allongé 2 lors de la phase de déplacement en retour, à l'état desserré, du mandrin mobile 5 (figures 1(c) et 1(d)).

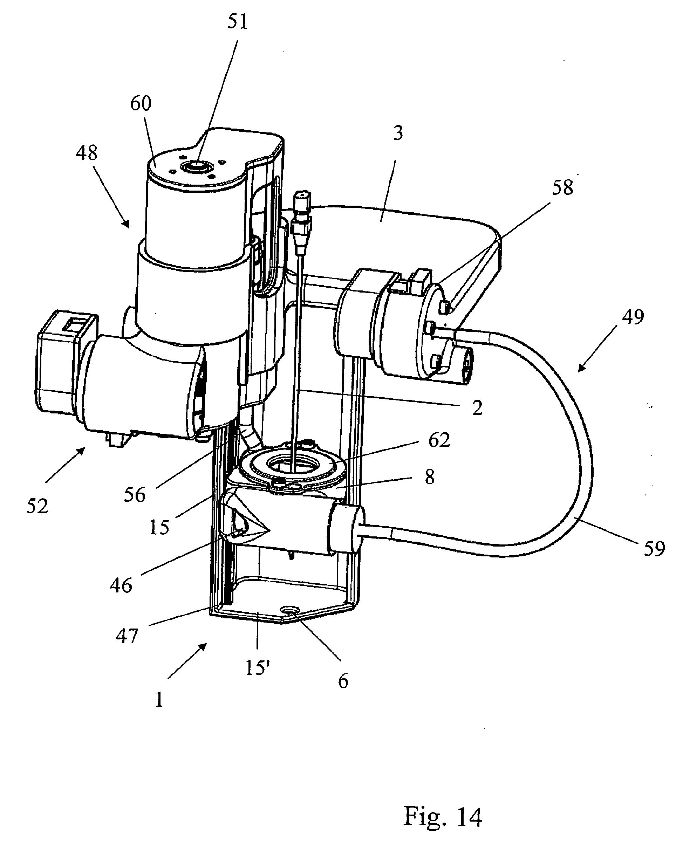

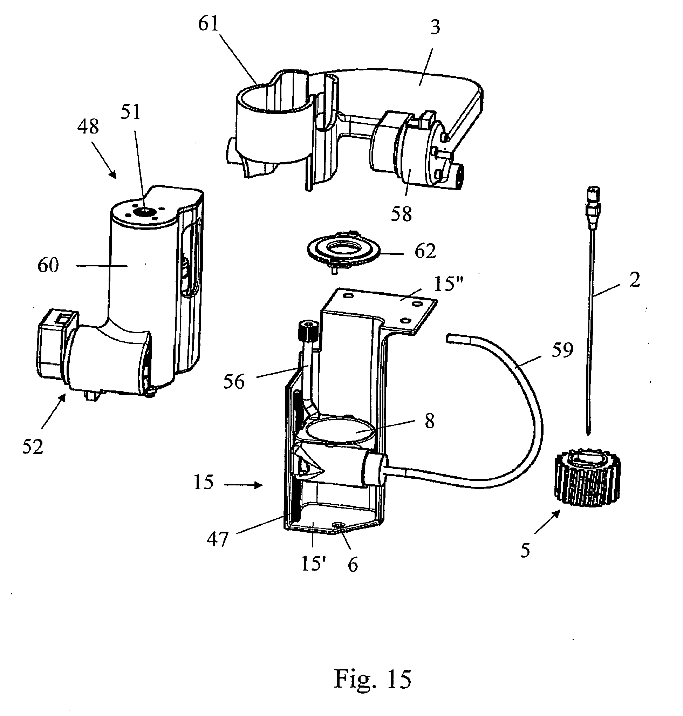

Selon une seconde variante de réalisation du second organe fixe 6, représentée aux figures 14 et 15 notamment, ledit second organe fixe 6 consiste en un orifice ou canal de guidage avec jeu du corps allongé 2, destiné à limiter le battement radial ou l'inclinaison angulaire du corps allongé 2 par rapport à la direction de translation Z et à maintenir ledit corps allongé 2 sensiblement dans une disposition coaxiale avec ladite direction Z, ce en coopération avec le mandrin 5 formant organe mobile de préhension et d'entraînement.

Le canal de guidage avec jeu peut par exemple consister en un canal effilé dans le sens positif de la direction Z, obtenu par déformation avec percement d'une paroi.

En accord avec une troisième variante de réalisation du second organe fixe 6, représentée aux figures 1, 2 et 3, le second organe fixe 6 réalise une fonction de préhension contrôlée du corps allongé 2 et consiste en un mandrin recevant le corps allongé 2 de manière traversante, ce second mandrin 6 présentant une structure, un mode de fonctionnement et des éléments constitutifs identiques à ceux du premier mandrin 5, à savoir notamment une couronne de manoeuvre 9', des mors 7' et un corps 10' avec un passage tubulaire 10".

En particulier en relation avec les première et troisième variantes précitées, il peut être avantageusement prévu de décomposer le déplacement translatif total envisagé en plusieurs déplacements de plus faible ampleur répétés de manière cyclique, le premier organe 5 mobile saisissant successivement le corps allongé 2 à des emplacements répartis le long de ce dernier. La figure 1 illustre schématiquement un tel cycle de déplacement (la croix sur l'organe 5 indiquant que ce dernier est en prise sur le corps 2).

Comme cela ressort de la figure 1, les deux mandrins 5 et 6 sont commandés en opposition de phase, c'est-à-dire que lorsque l'un d'entre eux est serré sur le corps 2, l'autre est desserré, et vice-versa.

Ainsi, durant les phases de translation effective et lorsque le mandrin 5 n'est pas dans une position extrême, c'est ce mandrin 5 qui serre et maintien le corps allongé 2. Le mandrin fixe 6 n'est donc serré que durant les phases de translation inverse du mandrin 5 non serré.

Afin d'éviter l'absence de toute prise sur le corps 2 et donc une possible incertitude sur la position ou le déplacement de ce dernier, il peut être prévu que les phases de serrage de deux mandrins 5 et 6 soient au moins légèrement chevauchantes, pour assurer au moins pendant un intervalle de temps court un contact simultané des deux mandrins 5 et 6 avec le corps 2.

L'utilisation de mandrins pour les organes 5 et 6 permet en outre de pouvoir manipuler, sans autre modification ou ajustement, des corps allongés 2 de diamètres différents (dans une plage de valeurs données, pour des corps 2 de section ronde), voire même des corps 2 de section polygonale.

De plus, les mors 7, 7' pourront comprendre des dents dont le profil assure une prise et un entraînement sûrs du corps allongé 2, notamment un blocage du corps 2 dans la direction du déplacement positif à l'état serré du mandrin considéré (en particulier le mandrin 5).

Ainsi, il peut être prévu de fractionner le mouvement de translation total du corps allongé 2 en une somme de translations élémentaires consécutives.

Comme cela ressort de la figure 1, le chariot 8 se trouve, dans un premier temps et en début d'une opération et d'une phase de translation, en position haute, le mandrin 5 étant alors serré sur le corps 2, tandis que les mors 7' du mandrin 6 sont tout juste en contact avec ledit corps 2. De cette manière, ce dernier mandrin 6 assure un simple guidage en translation du corps 2.

Le mouvement de translation élémentaire se décompose alors en quatre phases, telles qu'indiquées ci-après :

- descente du chariot 8 sur la longueur de la course élémentaire (figure 1a),
- lorsque le chariot 8 arrive en fin de course basse (figure 1b), le mandrin 5 est desserré, alors que le mandrin 6 est simultanément serré (figures 1b et 1c),
- le chariot 8 est alors ramené à sa position initiale, le mandrin 5 demeurant desserré (figure 1d),
- lorsque le mandrin 5 arrive en position initiale, il se serre alors que le mandrin 6 se desserre (figure le).

Bien entendu, le déplacement en translation du corps 2 dans le sens contraire sera réalisé en inversant simplement les opérations précitées.

Cette décomposition ou ce fractionnement du mouvement de translation total peut également être mis(e) en oeuvre lorsque le second organe fixe 6 est réalisé en accord avec sa seconde variante de réalisation précitée, en particulier lorsque le maintien du corps 2 à l'encontre de la pesanteur n'est pas nécessaire (par exemple aiguille 2 enfoncée dans un corps). Dans ce cas, l'organe mobile 5 agit comme décrit précédemment en relation avec la figure 1 et l'organe fixe 6 assure un maintien relatif (avec jeu) du corps 2 enfoncé.

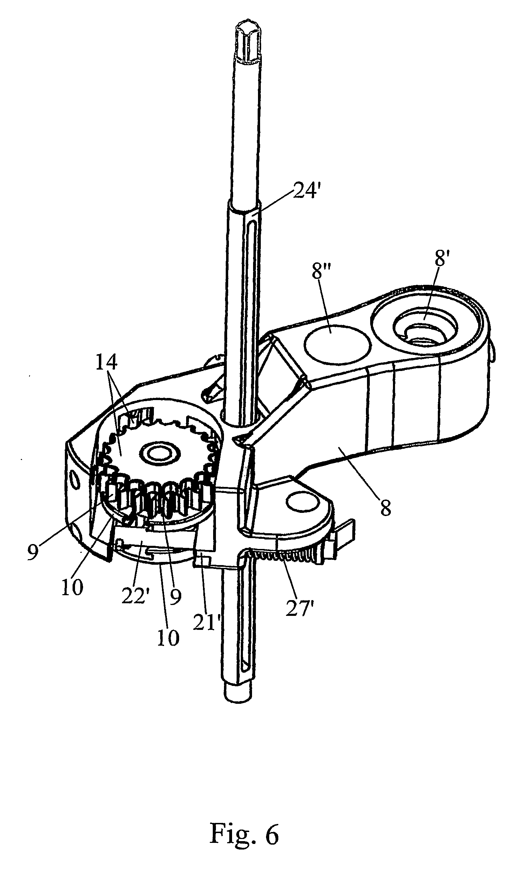

En relation avec les figures 1 à 9 des dessins annexés, on décrit ci-après plus précisément un premier mode de réalisation du dispositif 1 de maintien et de déplacement.

Comme le montrent les figures 8A et 8B, et selon une première variante de réalisation pratique possible, le ou chaque mandrin 5, 6 consiste essentiellement en un corps 10, 10' à passage traversant tubulaire 10", ouvert latéralement pour permettre le passage d'au moins deux, préférentiellement trois, mors 7, 7' et pourvu de moyens de guidage 11, 11' desdits mors 7, 7' dans une direction radiale par rapport au passage tubulaire du corps 10, 10' concerné. Lesdits mors 7, 7' sont répartis uniformément autour dudit passage 10" et peuvent être déplacés entre une position dite de serrage dans laquelle ils s'étendent partiellement dans le passage 10" correspondant et une position dans laquelle ils libèrent totalement ledit passage 10". De plus, lesdits mors 7, 7' comportent des segments de nervure / rainure 12 aptes à venir en engagement et à circuler dans une rainure / nervure 13 en portion de spirale ménagée dans une face latérale d'une couronne de manoeuvre dentée 9, 9' montée avec faculté de rotation sur une portion à forme externe tubulaire 10''' du corps 10, 10' du mandrin concerné 5, 6, une rotation de ladite couronne 9 ou 9' entraînant un coulissement radial simultané et identique des mors 7 ou 7'.

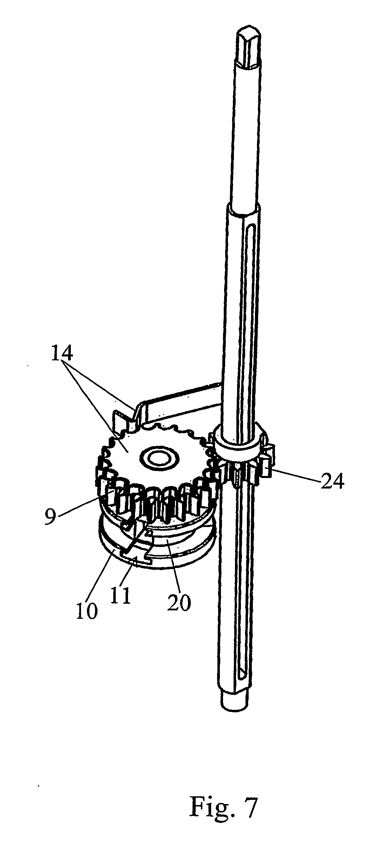

Conformément à une caractéristique additionnelle de l'invention, ressortant des figures 2 et 7 des figures annexées, le ou chaque mandrin 5, 6 est monté (dans le chariot 8 ou dans le site 16) avec faculté de rotation autour de l'axe longitudinal Z' du corps allongé 2 qui le traverse, le cas échéant, ensemble avec ce dernier, et à chaque mandrin 5, 6 est associé un mécanisme à cliquet 14, 14' bloquant le corps 10, 10' dudit mandrin 5, 6 concerné contre une rotation dans le sens du desserrage et autorisant une rotation progressive dudit mandrin 5, 6, dans le sens de serrage, lorsque ce dernier est intimement serré autour du corps allongé 2 et qu'un couple de serrage supplémentaire, présentant une intensité supérieure à une valeur déterminée définie par le mécanisme à cliquet 14, 14' concerné, est appliqué au niveau de la couronne de manoeuvre dentée 9, 9' correspondante.

Grâce à cette disposition, il est ainsi possible, non seulement de garantir un desserrage sûr, mais également de transmettre au corps allongé 2 un mouvement de rotation contrôlé et progressif (avec calage dans le sens de rotation inverse) autour de son axe longitudinal Z', ce en plus de son mouvement de translation.

Comme le montrent également les figures 2, 3 et 7, chacun des mécanismes à cliquet 14, 14' peut consister, par exemple, en une patte ou lame ressort avec un segment plié en forme de dent venant en engagement dans les dents ou crans d'un anneau ou d'une couronne solidaire du corps 10, 10' du mandrin concerné 5, 6.

La forme des dents et le degré de résistance à la déformation de la lame ressort détermineront la valeur du couple supplémentaire à appliquer pour obtenir une rotation du mandrin considéré.

Ainsi, les mécanismes à cliquet 14 et 14' permettent de bloquer la rotation des mandrins 5 et 6 respectivement dans un sens. Cette propriété est utile lors du desserrage desdits mandrins, pour empêcher la rotation du corps du mandrin correspondant 10, 10' lors de l'actionnement de la couronne 9, 9' concernée. Dans le sens du serrage, ces mécanismes à cliquet 14, 14' permettent aux mandrins 5 et 6 de tourner sur eux-mêmes au-delà d'un certain seuil de serrage du corps allongé 2, autorisant ainsi une rotation dudit corps 2 autour de son axe Z'.

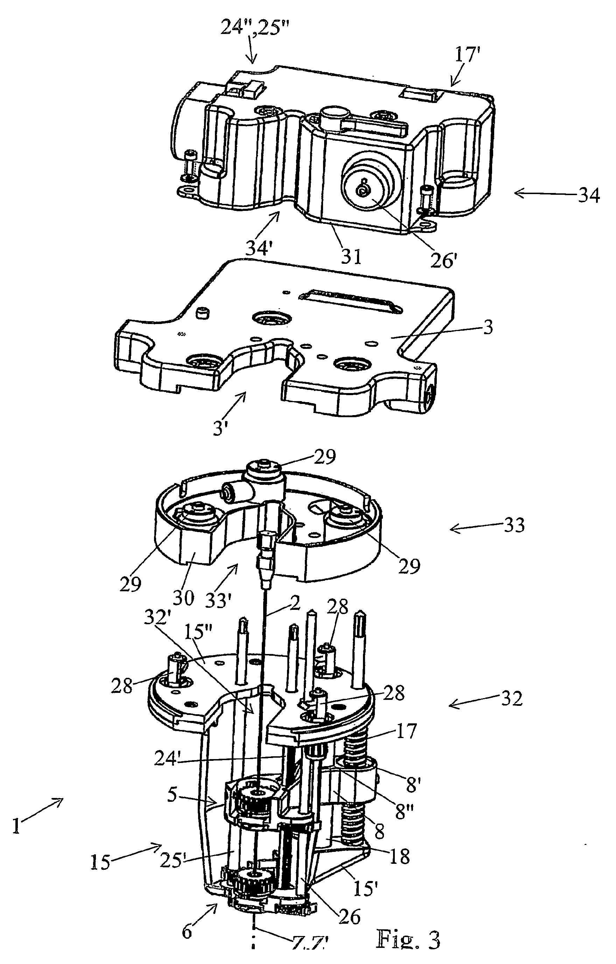

Comme le montrent également les figures 2 et 3, le premier organe mobile 5 et le second organe fixe 6 sont montés, préférentiellement de manière amovible, sur une structure support 15 apte à être assemblée rigidement avec la plateforme ou le bras 3 d'un dispositif robotique 4 de positionnement et d'orientation, la structure support 15 sous la forme d'un châssis présentant une extension donnée dans la direction de déplacement en translation Z du corps allongé 2, comportant un site de montage 16 pour le second organe fixe 6, positionné de manière extrémale dans le sens correspondant à un déplacement positif ou d'enfoncement dudit corps allongé 2 dans la direction de translation Z, et étant munie de moyens 17, 18 de guidage et de déplacement contrôlé du premier organe mobile 5 qui est monté, au niveau d'un site de montage 19 correspondant adapté, sur ou dans un chariot 8 coopérant avec ces moyens 17, 18.

En accord avec une réalisation avantageuse de l'invention, les moyens de guidage et de déplacement contrôlé du premier organe mobile 6 comprennent, d'une part, un arbre fileté 17 formant vis mère et, d'autre part, au moins un rail ou une tige de guidage 18. De plus, le chariot 8 intègre une perforation filetée 8' formant écrou fils et au moins un palier ou patin de guidage 8", traversant ou non, qui coopère, de manière coulissante, avec le ou chaque rail ou tige de guidage 18.

Préférentiellement, les premier et second organes 5 et 6, se présentant sous forme de mandrins, sont reçus de manière amovible et avec faculté de rotation dans leurs sites de montage respectifs 16 et 19 sensiblement en forme de U. En outre, les corps 10, 10' des mandrins 5, 6, de forme générale cylindrique circulaire, présentent une rainure circonférentielle externe 20, 20' et les sites de montage 16 et 19 présentent chacun une nervure 21, 21' apte à coopérer avec la rainure 20, 20' du corps 10, 10' de mandrin 5, 6 correspondant, sur une partie de leur extension circonférentielle, en vue de la rétention dudit corps 10, 10'. Enfin, lesdits sites de montage 16, 19 sont ouverts latéralement pour permettre la mise en place et l'enlèvement desdits mandrins 5, 6, ces derniers étant verrouillés à l'état monté dans leurs sites respectifs 16, 19 par des pièces de blocage mobiles 22, 22', préférentiellement sollicitées élastiquement en position de verrouillage desdits mandrins 5, 6, ces pièces de blocage 22, 22' venant en prise dans les rainures externes 20, 20' des corps 10, 10' des mandrins respectivement concernés 5, 6, dans la continuité des nervures 21, 21' et en fermant les ouvertures latérales 23, 23' des sites de montage respectifs 16, 19.

Dans certaines applications, en particulier médicales, il peut être nécessaire de disposer d'une possibilité de désengagement ou de désolidarisation rapide, et éventuellement automatique, du corps allongé d'avec le dispositif 1.

A cet effet, les premier et second organes 5 et 6 peuvent être montés dans leurs sites de montage 19 et 16 respectif à l'encontre d'une sollicitation élastique, cette dernière éjectant lesdits organes 5 et 6 hors desdits sites 19 et 16 lorsque les pièces de blocage 22, 22', préférentiellement commandées de manière simultanée, libèrent les ouvertures latérales 23 et 23' correspondantes.

Cette sollicitation élastique, peut par exemple être fournie par une lame ressort déformée par la mise en place du mandrin.

De manière alternative, il peut aussi être prévu que le chariot 8 portant l'organe de préhension 5 soit relié de manière amovible audit dispositif 1, en pouvant notamment être déplacé dans une position de coulissement extrême au niveau de laquelle il se désolidarise dudit dispositif 1.

Dans les deux variantes précitées, la désolidarisation s'effectue entre le dispositif 1 et les organes 5 et 6, ces derniers demeurant reliés au corps 2.

Afin de permettre notamment un déport des moyens d'actionnement des mandrins 5 et 6, tout en garantissant une commande fiable et robuste, l'entraînement de la couronne de manoeuvre 9, 9' de chaque organe en forme de mandrin 5, 6 peut être réalisé par l'intermédiaire d'un pignon 24, 25 engrenant avec ladite couronne 9, 9' et solidaire en rotation avec une tige ou un arbre 24', 25' entraîné(s) en rotation par un actionneur 24", 25" respectif correspondant, éventuellement communs. Le pignon 25 engrenant avec la couronne 9' du mandrin fixe 6 est solidarisé rigidement au niveau de l'extrémité d'un arbre d'entraînement 25' correspondant et en ce que le pignon 24 engrenant avec la couronne 9 du mandrin mobile 5 est solidaire en rotation et libre en translation par rapport à un arbre d'entraînement 24' correspondant (Fig. 7).

En outre, le dispositif 1 peut aussi comporter une tige ou tringle de commande 26 pivotante ou coulissante, reliée en entraînement avec les tiges de blocage 22, 22' en vue de leur déplacement hors de leur position de verrouillage à l'encontre de forces élastiques de sollicitation, par exemple fournies par des ressorts de compression 27, 27', cette tige de commande 26 étant solidaire en entraînement d'un mécanisme d'actionnement 26', en particulier un mécanisme de sécurité provoquant un déverrouillage par défaut.

Le mécanisme d'actionnement 26' peut par exemple consister en une ventouse électromagnétique qui maintient armée une lame de verrouillage rappelée par un ressort et solidaire de la tige de commande 26. En l'absence de courant, l'action de maintien de la ventouse disparaît et la lame de verrouillage pivote d'une fraction de tour en entraînant la tige de commande 26. Cette dernière entraîne alors le déplacement des pièces de blocage 22 et 22' et par conséquent le dégagement des mandrins 5 et 6.

Il convient de noter que l'une ou plusieurs des tiges 24', 25' et 26 précitées peuvent éventuellement servir de tige de guidage supplémentaire pour le chariot 8, au moins pour assurer un guidage non serré (absence d'interférence avec le mouvement rotatif de la tige considérée).

Lorsque le dispositif 1 est mis en oeuvre dans un environnement et en relation avec du matériel stérile, il peut être prévu que les arbres 24' et 25', ainsi que la tige de commande 26, présentent des extrémités libres, du côté des actionneurs 24", 25", respectivement du mécanisme 26', effilées ou en pointe, notamment aptes à percer la paroi d'un conditionnement stérile.

Dans l'exemple concret illustré sur les figures 2 et 3 notamment et en relation toujours avec le premier mode de réalisation du dispositif 1, le châssis formant structure support 15 comporte un plateau inférieur 15' renfermant le site de montage extrémal 16 pour le second organe 6 et un plateau supérieur 15" de fixation à la plateforme 3 d'un dispositif robotique 4 de fonctionnement et d'orientation, ledit plateau supérieur 15" étant pourvu de moyens d'assemblage rapide 28, tels que par exemple des loquets, aptes à venir en prise avec ladite plateforme 3 ou avec une pièce solidaire de cette dernière à l'état monté dudit châssis 15. Les moyens 17, 18 de guidage et de déplacement contrôlé, ainsi que le cas échéant les arbres d'entraînement 24', 25' et la tige de commande 26, s'étendent et sont montés entre ces deux plateaux 15' et 15" dont l'espacement mutuel détermine sensiblement la course du premier organe mobile 5.

Le plateau inférieur 15 comportera des paliers pour les tiges de commande 24', 25', 26 et l'arbre 17, le plateau supérieur 15" comportant des orifices traversants, formant éventuellement également paliers.

Les deux plateaux 15' et 15" peuvent par exemple être reliés par des entretoises ou une ou plusieurs portions de paroi, ces derniers éléments pouvant éventuellement fournir des rails ou des tiges de guidage supplémentaires pour le chariot 8.

Afin de disposer d'informations sur le déplacement du corps 2 en plus de sa position et de pouvoir le cas échéant mettre en place des mesures de sécurité, le dispositif peut comprendre un ou des capteurs d'effort 29, 54' mesurant l'intensité de la poussée exercée sur le corps allongé 2 dans la direction de translation Z, et le cas échéant du couple ou de certaines composantes au moins du couple exercé sur ce corps allongé 2.

Selon un mode de construction et de montage avantageux du dispositif 1, lesdits capteurs d'effort 29 sont disposés entre le plateau supérieur 15" de la structure support 15 et la plateforme 3 du dispositif robotique 4, et étant préférentiellement montés sur un plateau 30 intercalaire pris en sandwich entre ledit plateau supérieur 15" et ladite plateforme 3.

Dans cette réalisation, et pour assurer une transmission complète des efforts / sollicitations exercé(e)s sur le corps 2 aux capteurs 29, les loquets 28 viennent en prise sur ou dans le plateau 30, ce dernier étant fixé sur la plateforme 3.

Conformément à l'exemple représenté sur la figure 3 et repris schématiquement sur les figures 4A et 4B, les capteurs d'effort 29 consistent en des capteurs unidirectionnel orientés dans la direction de translation Z, sont au nombre de trois et sont disposés dans un plan orthogonal à ladite direction 2 et positionnés de manière symétrique sur un cercle de rayon r centré sur l'axe longitudinal Z' du corps allongé 2, cet arrangement de trois capteurs 29 autorisant ainsi la détermination de deux composantes du couple de réaction exercé sur le corps allongé 2.

Sur les figures 4A et 4B, les trois capteurs 29 sont différenciés par les désignations supplémentaires $A_1$, $A_2$, $A_3$. Le point B désigne, dans le cadre d'une procédure d'enfoncement du corps allongé 2, le point d'entrée, ce dernier étant localisé à une distance d du plan formé par les trois capteurs 29.

La composante Z de la force F exercée par le corps dans lequel le corps allongé 2 est introduit peut être calculé à partir des forces $m_1$, $m_2$ et $m_3$ mesurées par les trois capteurs comme suit :

$$\vec{F}.\vec{z} = Mg\vec{z_0}.\vec{z} - m_1 - m_2 - m_3.$$

Dans cette formule $z$ indique l'axe du corps allongé 2, $z_0$ indique la direction de la pesanteur, M est la masse du dispositif 1 et $- gz_0$ correspond à l'accélération de la gravité.

La position et l'orientation de la plateforme 3 demeurant inchangées durant le déplacement du corps allongé,

Mgz₀.z constitue une valeur déterminable en début de procédure.

L'arrangement spécifique des capteurs unidirectionnels 29 permet de calculer deux composantes du couple exercé sur l'aiguille, exprimées au point O :

$$\vec{M}.\vec{x} = Mg\overrightarrow{OG} \wedge \vec{z_0}.\vec{x} - \underline{\frac{r}{2}} \, m_2 - \underline{\frac{r\sqrt{3}}{2}} \, m_3.$$

$$\vec{M}.\vec{y} = Mg\overrightarrow{OG} \wedge \vec{z_0}.\vec{y} + rm_1 - \underline{\frac{r\sqrt{3}}{2}} \, m_2 - \underline{\frac{r}{2}} \, m_3.$$

Dans ces deux formules G indique le centre de masse du dispositif 1.

Bien entendu, les capteurs 29 peuvent également consister en des capteurs multidirectionnels permettant de fournir des mesures sur le couple et les forces axiales.

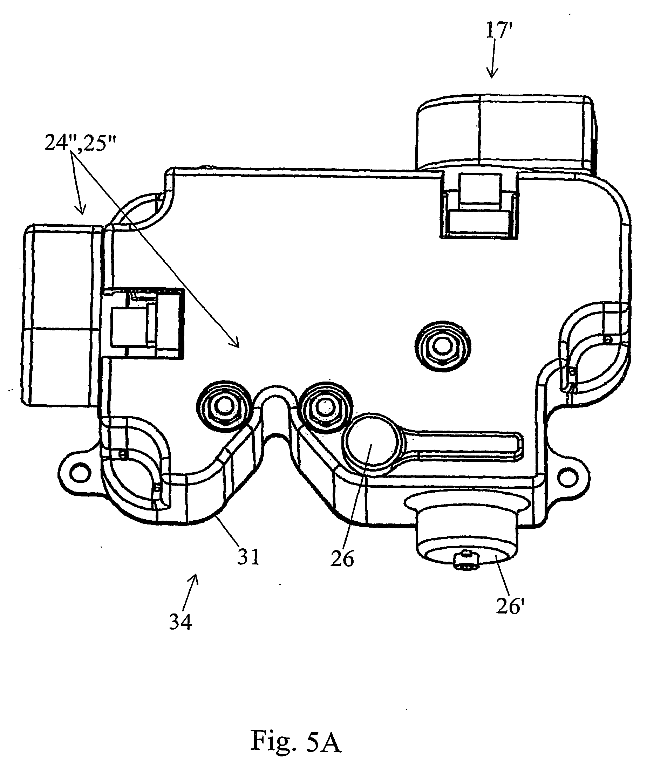

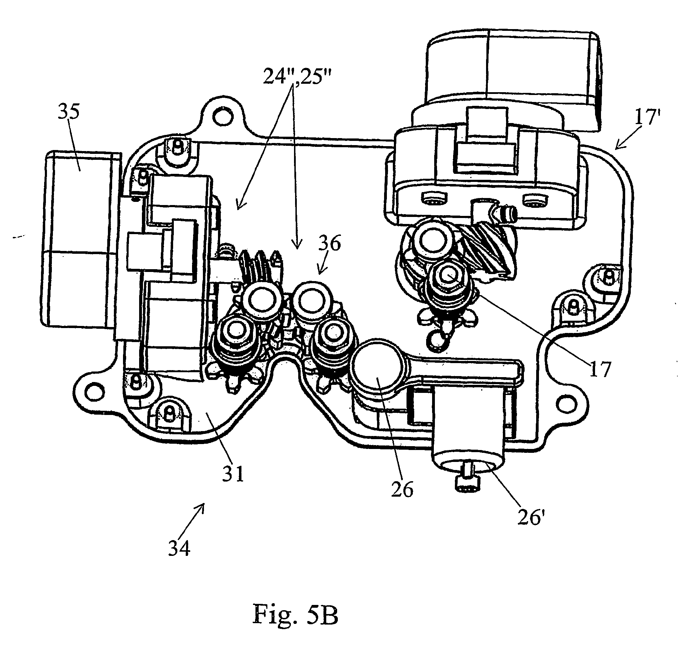

En vue de contribuer à une construction d'ensemble simple et compacte du dispositif 1, les actionneurs 24", 25" entraînant les arbres 24', 25' sont regroupés sur un plateau support 31 rapporté sur la plateforme 3 du dispositif robotique 4 à l'opposé de la structure support 15 portant les premier et second organes 5 et 6, ce plateau support 31 portant, le cas échéant, également le mécanisme d'actionnement 26' de la tige de commande 26 apte à déplacer les pièces de blocage 22, 22' hors de leur position verrouillée (Fig. 5A et 5B).

Le plateau 31 peut, par exemple, être relié au plateau supérieur 15" de la structure 15 par des vis, qui traversent également les plateaux 30 et la plateforme 3, en assemblant ainsi ces différents éléments entre eux.

En accord avec un perfectionnement avantageux de l'invention, permettant de simplifier, d'alléger et de réduire le coût du dispositif 1, il peut être prévu que les actionneurs 24", 25" des arbres 24', 25' se présentent sous la forme d'un actionneur double intégrant un unique moteur 35 à couple élevé et à faible vitesse de rotation, par exemple du type piézoélectrique, et un arrangement 36 d'engrenages de transmission transformant le mouvement de rotation de sortie dudit moteur en deux mouvements de rotation synchrones et de sens opposés, fournis aux deux arbres 24' et 25'.

Comme le montrent également les figures 5A et 5B, le plateau support 31 porte également un actionneur 17' pour l'arbre fileté 17 déplaçant le chariot 8, ce par exemple sous la forme d'un moteur piézoélectrique associé à un mécanisme d'engrenage.

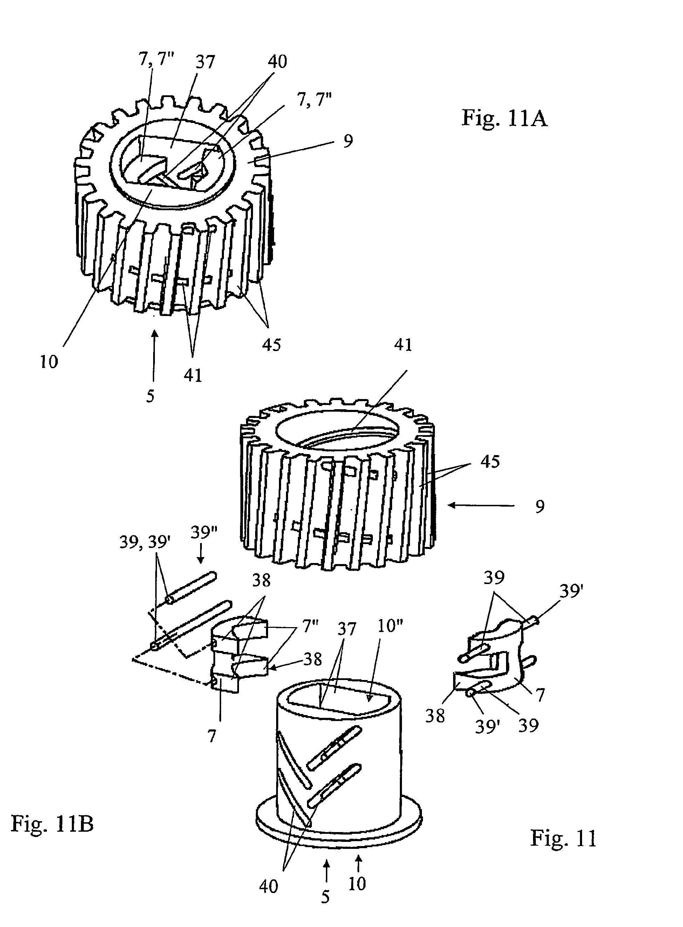

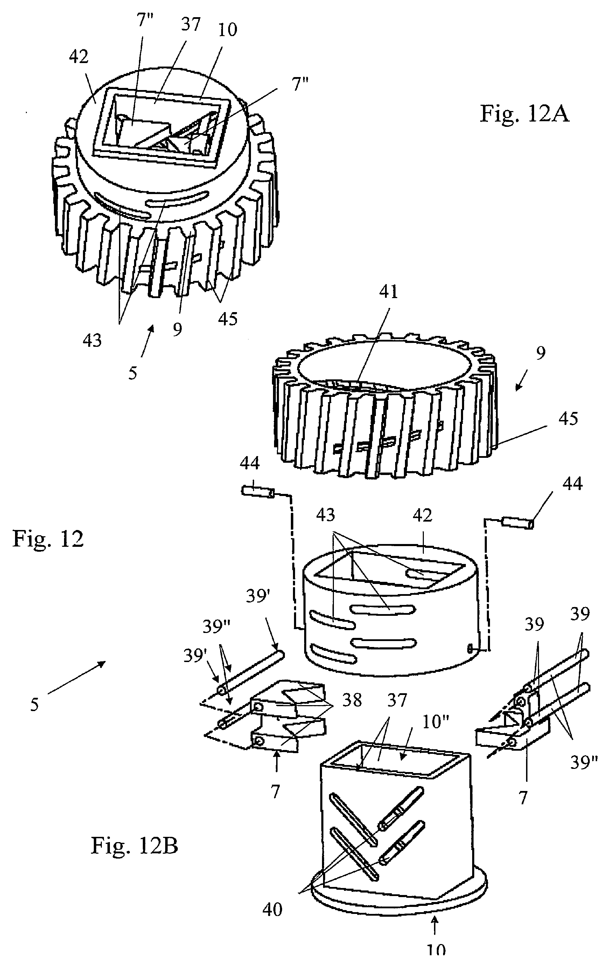

En accord avec d'autres formes de réalisation du mandrin 5, représentées aux figures 11 et 12, les mors 7 peuvent être montés guidés dans le passage traversant tubulaire 10" du corps 10, lesdits mors 7 étant pourvus de moyens d'entraînement 39 sollicités, directement ou indirectement, par la couronne de manoeuvre 9 entourante, de manière à être déplacés en translation selon Z ou Z' lors de la rotation de cette couronne 9, en générant un mouvement simultané de rapprochement ou d'éloignement mutuel desdits mors 7 autour de l'axe médian dudit passage 10" ou d'un corps allongé 2 monté dans ledit mandrin 5.

Plus précisément, les moyens de serrage 7 peuvent consister en deux mors opposés et en regard, avec des mâchoires 7" mutuellement décalées de manière à s'interpénétrer lors de leur mouvement de rapprochement en vue du serrage. Le passage tubulaire 10" présente alors deux faces opposées planes 37 formant surfaces de guidage plan pour lesdits mors 7, ces derniers étant également pourvus de portions de surfaces de guidage latérales 38 adaptées pour glisser en appui sur lesdites faces 37. Chacun desdits mors 7 est guidé de manière supplémentaire durant leurs mouvements de rapprochement et d'écartement mutuel pour effectuer simultanément un déplacement dans la direction Z ou Z'.

En accord avec une forme de réalisation concrète du guidage et de l'entraînement des mors 7, chacun desdits mors 7 peut être pourvu d'au moins deux tiges d'entraînement et de guidage 39" saillantes latéralement par rapport à ses portions de surfaces de guidage 38 et formant chacune une paire d'ergots ou de doigts 39 opposés, ces derniers étant, d'une part, montés coulissant dans des portions de rainures 40 ménagées dans les portions de paroi du corps 10 définissant les faces opposées planes 37, coïncidentes deux par deux en cas de superposition des deux faces opposées planes 37 et situées par paires coïncidentes dans des plans ou des surfaces non planes incliné(e)s par rapport à la direction longitudinale Z' d'un corps allongé 2 monté de manière serrée dans le mandrin 5 et, d'autre part, au moins pour une paire de doigts ou d'ergots 39 opposés de chaque mors 7, en relation d'entraînement, directe ou indirecte, avec la couronne de manoeuvre 9 au niveau de leurs extrémités 39' dépassant des portions de rainures 40 qu'ils traversent, en vue de leur déplacement dans la direction longitudinale Z' lors d'un mouvement de rotation de ladite couronne de manoeuvre 9.

Les figures 11 et 12 montrent les tiges 39" à l'état démonté et à l'état monté (Fig. 11B) dans des orifices traversants des mors 7, les parties dépassant de ces derniers formant les doigts ou ergots 39.

En prévoyant des portions de rainures 40 inclinées par rapport à la direction Z (c'est-à-dire non compris dans un plan perpendiculaire à cette direction), il est possible de fournir un dégagement ou une course radial(e) de grande ampleur pour les mors 7 du fait d'un déplacement guidé combiné avec simultanément une composante de déplacement de coulissement dans un plan perpendiculaire à la direction Z et une composante de déplacement en translation selon cette dernière direction.

Les portions de rainures 40 présentent une forme rectiligne sur les figures 11 et 12 et engendrent de ce fait une proportionnalité constante entre les deux composantes de déplacement tout au long de la course des mors 7.

Néanmoins, d'autre forme de portions de rainures 40, non linéaires, sont possibles, par exemple pour disposer d'un couple de serrage plus important en fin de course de rapprochement des deux mors 7.

Grâce notamment à ce mouvement combiné des mors 7, associé à la constitution particulièrement compacte du mandrin 5, le rapport entre l'ouverture interne maximale du mandrin 5, pour un écartement maximal des mors 7, et le diamètre externe de la couronne 9 peut être obtenu supérieur à 0,2, préférentiellement d'au moins 0,3.

Un tel degré d'ouverture permet de garantir un relâchement total du corps 2 à l'état ouvert (plus aucun engagement des mors 7 avec le corps 2, mais seulement un maintien latéral avec battement dans le passage tubulaire 10") et une grande plage d'adaptation du mandrin 5 à différents diamètre de corps allongés 2.

Selon une première variante pratique, ressortant des figures 11A et 11B, le corps 10 peut présenter une forme extérieure cylindrique circulaire, ajustée en diamètre au diamètre intérieur de la couronne de manoeuvre 9, les extrémités 39' des doigts ou ergots 39 qui dépassent des portions de rainure 40 au niveau de la face externe du corps 10 venant en engagement glissant guidé dans des rainures 41 ménagées au niveau de la face interne de la couronne de manoeuvre 9 et formées par des portions de courbes engendrées chacune par l'intersection entre une surface réglée déterminée et la surface cylindrique interne de ladite couronne 9, le corps 10 étant bloqué en translation et libre en rotation dans cette couronne 9 entourante.

Chaque mors 7 possède alors au moins deux paires opposées d'ergots ou de doigts 39, à savoir, une première paire plus longue qui vient en engagement avec la couronne 9 et assure ainsi l'entraînement dudit mors 7, et au moins une seconde paire plus courte qui ne vient pas en engagement avec la couronne 9 et contribue ainsi uniquement au déplacement guidé dudit mors 7, en coopération avec la première paire.

A titre d'exemple, on exploite ci-après l'équation des courbes permettant de générer les découpes (rainures 41) dans la couronne 9 du mandrin 5 de la figure 11.

Si on note $(O, e_r, e_\theta, e_z)$ un repère cylindrique,

si on appelle C, la surface cylindrique intérieure de la couronne 9, d'axe $(O, e_z)$ et de rayon R et

si on note D la droite support de l'axe d'un des doigts ou ergots 39,

alors D, initialement dans le plan $(O, e_r, e_\theta)$, se déplace en un mouvement combiné :

- de translation suivant $(O, e_z)$ et suivant une direction radiale $(O\ e_r)$ ;
- de rotation autour de $(O, e_z)$.

Une rainure 41 est obtenue à partir des courbes intersections de D et de C.

Les équations paramétriques de ces courbes, dans le repère cylindrique $(O, e_r, e_\theta, e_z)$, sont les suivantes :

pour t variant de 0 à 1 :

$$\begin{cases} r(t) = R \\ \theta(t) = \alpha(t) + Arc\cos(\dfrac{e(t)}{R}) \\ z(t) = f_1(t) \end{cases}$$

et

$$\begin{cases} r(t) = R \\ \theta(t) = \alpha(t) - Arc\cos(\dfrac{e(t)}{R}) \\ z(t) = f_1(t) \end{cases}$$

Pour la réalisation particulière proposée dans le cadre de l'invention et illustrée sur la figure 11, $\alpha$, $e$ et $f_1$ sont des fonctions linéaires du temps.

Selon une seconde variante pratique, ressortant des figures 12A et 12B, le corps 10 peut présenter une forme extérieure carrée ou rectangulaire et être monté dans la couronne de manoeuvre 9 avec interposition d'une pièce intermédiaire 42 présentant une forme extérieure cylindrique circulaire, ajustée en diamètre au diamètre intérieur de la couronne 9 et ayant une forme intérieure complémentaire de la forme extérieure du corps 10, cette pièce intermédiaire 42 étant pourvue, d'une part, de rainures 43 coïncidentes par paires, disposées par paires dans des plans perpendiculaires à l'axe médian du passage tubulaire 10" et recevant, avec un engagement coulissant guidé, les extrémités 39' des doigts ou ergots 39 qui dépassent des portions de rainures 40 au niveau de la face externe du corps 10 et, d'autre part, d'au moins deux ergots 44 saillants au niveau de sa face extérieure et venant en engagement glissant guidé dans des rainures 41 sensiblement en forme de portions d'hélices et ménagées au niveau de la face interne de la couronne de manoeuvre 9, le corps 10 étant bloqué en translation et libre en rotation dans cette couronne 9 entourante.

De manière avantageuse, la couronne de manoeuvre 9 présente sur sa face extérieure périphérique une denture inclinée 45, adaptée à un engagement fonctionnel (engrènement) avec une vis sans fin 46 en vue de son entraînement en rotation, un entraînement de ladite couronne 9 au-delà d'un degré de serrage déterminé du corps allongé 2 par les mors 7, provoquant une rotation du mandrin 5 et du corps 2 serré.

Ainsi, le corps 2 peut être entraîné et positionné en rotation de manière contrôlée autour de son axe Z', l'engrènement mutuel de la couronne 5 et de la vis 46 garantissant une cinématique exempt de jeu.

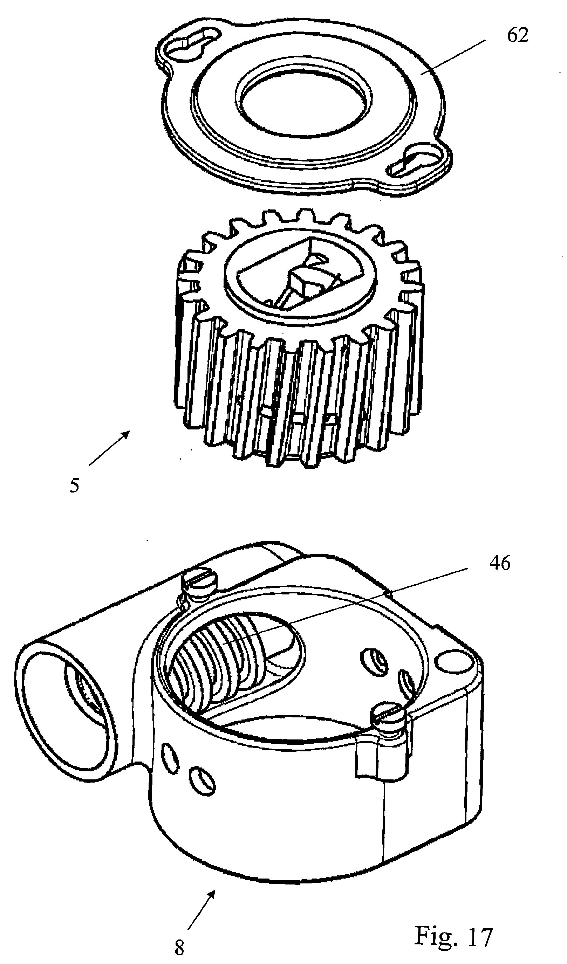

Conformément à un autre mode de réalisation avantageux de l'invention, ressortant en particulier des figures 14 à 17, le mandrin 5 est assujetti, de manière amovible et avec faculté de rotation, dans un chariot 8 monté coulissant dans la direction de translation Z dans la structure support allongé 15 munie d'au moins une glissière 47 adaptée, ladite structure 15 étant pourvue d'un plateau supérieur 15" pour sa fixation sur la plateforme 3 du dispositif robotique 4 et d'un plateau inférieur 15' comportant un orifice ou canal de guidage avec jeu et de centrage formant le second organe fixe 6. Avantageusement, un ensemble motorisé 48 de déplacement contrôlé du chariot 8 et un ensemble motorisé 49 d'actionnement de la couronne de manoeuvre 9 sont associés à l'ensemble chariot 8 / corps support 15, en étant montés soit sur la plateforme 3, soit sur ledit corps support 15.

L'assujettissement du mandrin 5 dans le chariot 8, avec faculté de rotation autour de la direction Z, peut par exemple être obtenu en fournissant dans le chariot 8 un logement annulaire de forme adaptée avec une ouverture d'introduction obturable (en conservant un passage pour le corps 2 traversant) par un couvercle 62 amovible (également de forme annulaire).

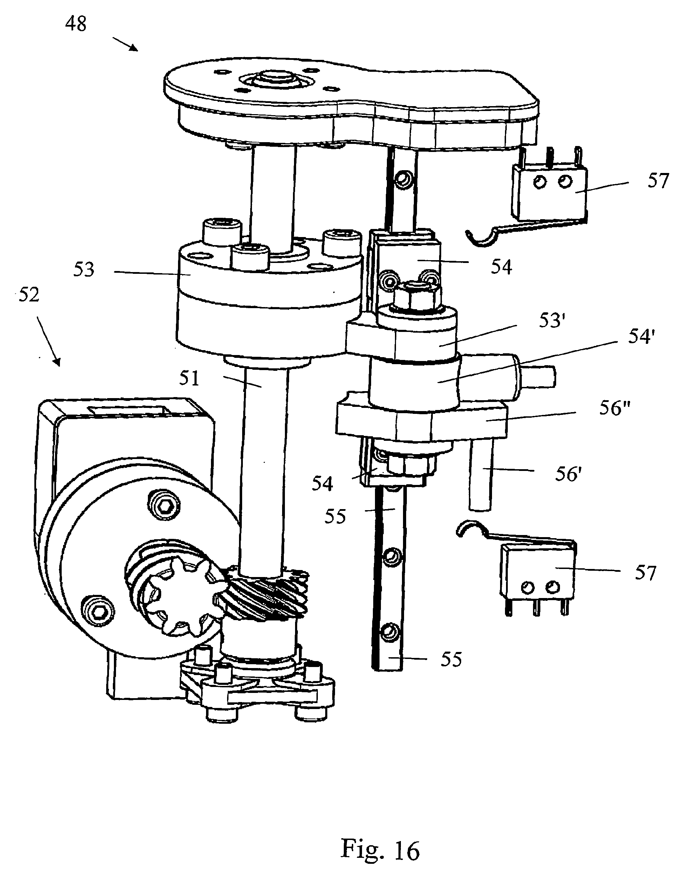

Comme le montrent les figures 15 et 16, l'ensemble d'entraînement motorisé 48 peut par exemple consister en une vis mère 51 entraînée en rotation par un bloc-moteur 52 associé éventuellement à un codeur de position, et entraînant en translation un écrou fils 53, ledit écrou fils 53 étant relié rigidement à un chariot ou une paire de chariots de guidage intermédiaire(s) 54 coulissant sur un rail 55 parallèle à la direction de translation Z et à la glissière 47. Ledit ou lesdits chariot(s) 54 est (sont) également relié(s) rigidement à un site 56' d'accouplement amovible d'une barre de liaison 56 solidaire du chariot 8. Les points de fixation et de liaison dudit écrou fils 53 et dudit site d'accouplement 56' avec le ou l'un des chariots 54 étant reliés entre eux avec interposition d'un capteur d'effort 54' apte à mesurer l'intensité des actions mécaniques dans la direction Z, et ledit dispositif 1 comporte en outre des fins de course 57 opposés, mutuellement décalés dans la direction de translation Z, et délimitant la course maximale dudit ou desdits chariot(s) intermédiaire(s) 54 et donc du chariot 8, ledit ensemble motorisé 48, à l'exception des fins de course 57, étant éventuellement monté dans un carter 60, formés d'un seul tenant avec la plateforme 3 ou reçu de manière indexée et avec blocage en position, dans un site de réception 61 solidaire de la plateforme 3, en formant ainsi un module autonome amovible.

Comme le montrent les figures 14 et 15, l'ensemble d'actionnement motorisé 49 peut consister en un bloc-moteur 58 entraînant, par l'intermédiaire d'un arbre de transmission flexible 59, une vis sans fin 46 intégrée au chariot 8 étant en engagement d'entraînement avec la couronne de manoeuvre 9 périphérique du mandrin 5 monté dans ledit chariot 8, ledit bloc-moteur 58 étant monté dans un carter formé d'un seul tenant avec la plateforme 3 ou reçu de manière indexée et avec blocage en position dans un site de réception de ladite plateforme 3.

Le capteur d'efforts 54' réalise une fonction similaire à celle des trois capteurs 29, sauf à ne mesurer que les efforts selon la direction Z.

Comme le montre la figure 16, le capteur d'efforts 54' peut être pris en sandwich entre une bride d'entraînement 53' solidaire de l'écrou fils 53 et une bride d'entraînement 56" solidaire du site d'accouplement 56', l'ensemble [53'/54'/56"] étant monté rigidement entre les deux chariots 54 (Fig. 16).

Les blocs-moteurs 52 et 58 sont préférentiellement du type à blocage en cas de défaut ou d'absence d'alimentation et à mouvement contrôlé par capteur.

Bien entendu, l'invention décrite ci-dessus, en relation avec trois modes de réalisation différents, peut être mise en oeuvre dans un grand nombre d'applications et de domaines.

Toutefois, dans le cadre d'une application préférée de l'invention, le corps allongé 2 consiste en une aiguille pour la réalisation de procédures médicales percutanées.

De telles aiguilles ont généralement un diamètre inférieur au millimètre ou de plusieurs millimètres, et le dispositif 1 présentera un mandrin 5, et le cas échéant un mandrin 6, apte(s) à recevoir ces différents diamètres et aptes à transmettre une force de poussée avantageusement d'au moins 10N, préférentiellement d'environ 15N.

On notera que, dans le cadre d'une telle application médicale notamment, les résultats des mesures des capteurs 29 peuvent être affichées, ou reproduites sous forme d'efforts résistants ou de sollicitations contraires au niveau d'un organe de commande manipulé par l'opérateur du dispositif 1.

Cette dernière variante permet ainsi au praticien de percevoir les transitions du passage de l'aiguille 2 au travers des différentes structures anatomiques.

Ainsi que le montrent les figures 3, 13, 15 et 18 des dessins annexés, la plateforme 3 du dispositif robotique 4 présente une découpe ouverte 3', de même qu'éventuellement le plateau supérieur 15" de la structure support 15 (elle-même ajourée latéralement), la plaque de montage intermédiaire 50, le plateau intercalaire 30 portant les capteurs d'effort 29 et le plateau support 31 des actionneurs 17', 24', 25', l'ouverture de cette ou ces découpe(s) étant orientée dans la même direction que les ouvertures 23, 23' des sites de montage 16 et 19 des premier et second organes 5 et 6 et/ou qu'un dégagement latéral de la structure support 15, de manière à permettre une mise en place et/ou un enlèvement latéral(e) de ladite aiguille 2.

La découpe 3', associée, le cas échéant, à la découpe 32' du plateau 15", à la découpe 33' du plateau intercalaire 30 et à la découpe 34' du plateau support 31 permet ainsi d'avoir une possibilité d'engagement et de dégagement latéraux (par rapport aux directions axiales Z, Z') du corps allongé 2, par exemple sous forme d'aiguille. Lorsque les organes 5 et 6 sont libérés de leurs sites de montage respectifs 19 et 16, il est ainsi possible de désolidariser et d'extraire rapidement le corps 2 du dispositif 1, sans nécessiter de manipulations ou de procédures d'extraction longitudinale complexes et coûteuses en temps. Ceci est également possible lorsque seul l'organe 5 peut être libéré.

Comme déjà indiqué précédemment, et en relation avec le premier mode de réalisation, la mise en place et l'enlèvement de l'aiguille 2 s'effectueront ensemble avec les mandrins 5 et 6, lors de la mise en place l'un de ces derniers étant serré et l'autre desserré.

Dans les modes de réalisation des figures 13 à 15, le mandrin 5 sera également préférentiellement monté serré sur l'aiguille 2 avant son montage dans le chariot 8 ou son introduction dans le corps support 15.

La présente invention a également pour objet un système d'assistance aux procédures médicales percutanées guidées par imagerie médicale, comprenant un dispositif robotique de positionnement et d'orientation d'une plateforme, un dispositif d'insertion et de rétraction contrôlées d'aiguille et un dispositif de pilotage desdits dispositifs en fonction de paramètres préprogrammés et de résultats de mesures instantanées, telles que des mesures extraites d'images médicales peropératoires, des mesures de sollicitations dans la direction axiale de l'aiguille et/ou des sollicitations de couple au niveau de ladite aiguille, ou encore des indications ou mesures de position fournies par le dispositif robotique et/ou le dispositif d'insertion précités. Ce système est principalement caractérisé en ce que le dispositif d'insertion et de rétraction d'aiguille consiste en un dispositif 1 tel que décrit ci-dessus, en relation avec l'un quelconque de ses modes de réalisation.

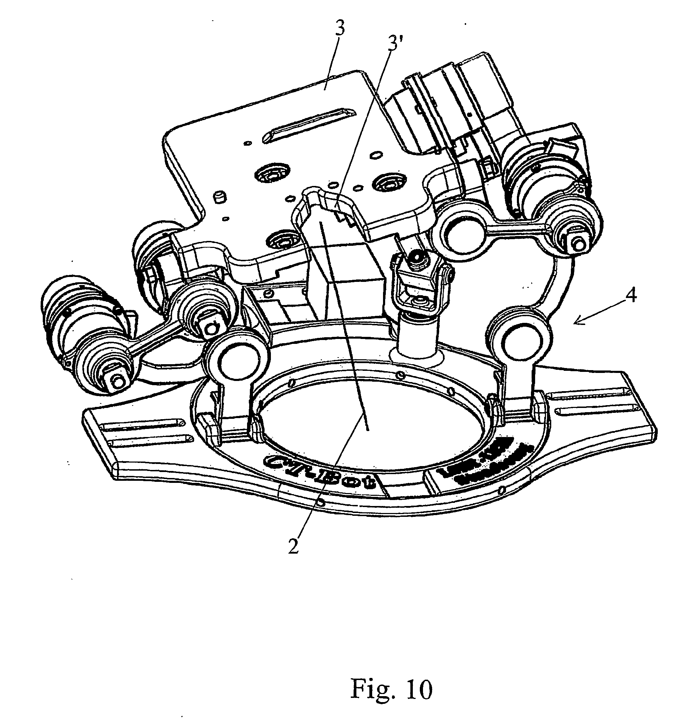

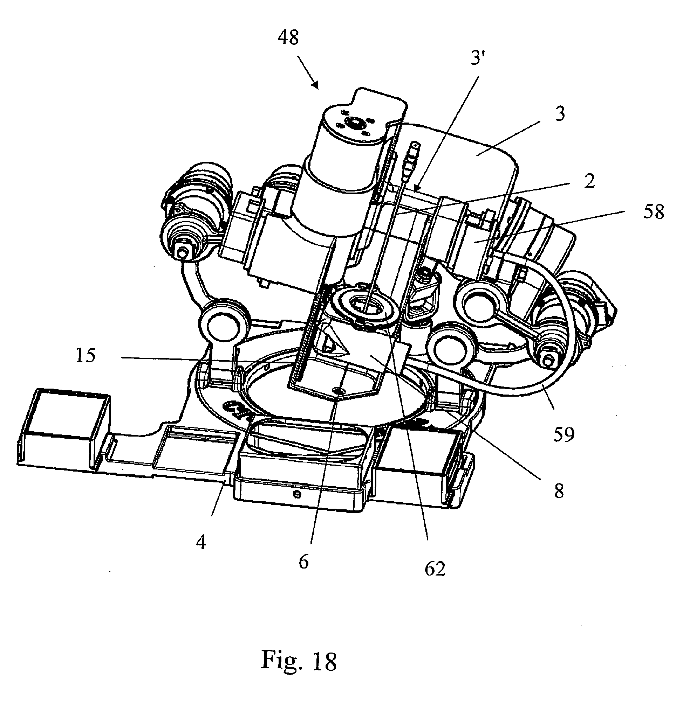

Une construction possible d'un tel système peut être obtenu en associant les objets représentés sur les figures 3 et 10 (sans le dispositif de pilotage). Une autre construction possible est représentée sur la figure 18.

Le dispositif robotique 4 peut notamment être du type de celui décrit dans :

"A parallel 5 dof positionner for semi-spherical workspaces" ("Positionneur à cinq degrés de liberté et à structure parallèle pour des volumes de travail semi-sphériques") de B. Maurin et al., Proceedings of the 2004 Asme Design Engineering Technical Conferences, DETC'04 ; ou dans la demande de brevet PCT n° PCT/FR2005/002357 au nom des demandeurs.

De manière avantageuse, et comme le montre la figure 3 des dessins annexés, en relation avec un premier mode de réalisation, le dispositif 1 d'insertion et d'extraction d'aiguille 2 peut présenter une structure modulaire étagée, avec un premier module 32 renfermant les premier et second organes 5 et 6 et les moyens de commande mécaniques de ces derniers, un second module 33 renfermant des moyens capteurs, et un troisième module 34 renfermant les moyens d'actionnement des moyens de commande mécaniques précités, les second et troisième modules 33 et 34 étant conditionnés dans un emballage stérile, ensemble avec une partie attenante au moins du dispositif robotique 4 de positionnement et d'orientation, et le premier module 32 étant réalisé, partiellement ou totalement, en tant que module du type consommable ou à usage unique.

Ainsi, il peut être procédé, à chaque nouvelle insertion d'aiguille 2, au remplacement de la totalité du premier module 32 ou d'une partie seulement de ce dernier (par exemple des seuls mandrins 5 et 6), ce en fonction notamment du coût de revient du module et/ou de sa possibilité de résister aux traitements de stérilisation.

En relation avec les autres modes de réalisation de l'invention, représentés aux figures 13 à 18, il peut être prévu en variante que le corps support 15, le cas échéant avec le chariot 8 et éventuellement le flexible 59, forme(nt) un module du type consommable ou à usage unique (de même que le mandrin 5 et l'aiguille 2), la partie complémentaire du système (système robotique 4, plateforme 3, dispositifs 48 et 49) étant conditionnée dans un emballage stérile (avant chaque utilisation).

La structure support 15, le(s) mandrin(s) 5 (et 6) et éventuellement certains des arbres ou tiges de commande peuvent être réalisés en un matériau polymère résistant et aisés à moulé, le cas échéant renforcé par des fibres.

Comme cela ressort de la description qui précède, le dispositif 1, en particulier sous la forme d'un dispositif d'insertion et d'extraction d'aiguilles, et en fonction de son mode de réalisation décrit ci-dessus, présente différentes propriétés de sécurité.

Ainsi, la préhension de l'aiguille 2 et son déplacement sont totalement découplés d'un point de vue cinématique et sont réalisés par l'intermédiaire de mécanismes de transmission unidirectionnels. De ce fait, le contrôle du dispositif 1 est simplifié et la position de la pointe de l'aiguille 2 n'est pas influencée par son interaction avec l'environnement.

De plus, l'utilisation de moteurs piézoélectriques ou ultrasonores contribue également à la sécurité du dispositif 1 dans le cas d'une coupure d'alimentation, puisque la configuration et la position d'entraînement de l'aiguille 2 demeurent inchangées.

Enfin, les mandrins reliés à l'aiguille peuvent être extraits à tout moment durant les phases d'insertion et d'extraction, grâce au mécanisme de déblocage de sécurité 22, 22', 26, 26'.

En ce qui concerne les exigences en matière de stérilité, on peut noter que le dispositif 1 peut se subdiviser en deux parties, à savoir, une partie active comprenant les actionneurs et les capteurs et qui est reliée à la plateforme 3 (cet ensemble peut être conditionné dans un emballage stérile) et une partie passive ou purement mécanique intégrant la structure 15, les arbres et tiges de commande et les mandrins (cette partie étant jetable et/ou stérilisable).

Par ailleurs, on notera que les matériaux constitutifs des différentes composantes du dispositif 1 sont avantageusement compatibles avec le type d'imagerie médicale utilisée, par exemple scannographe (en anglais "CT-scan").

Bien entendu, l'invention n'est pas limitée au mode de réalisation décrit et représenté aux dessins annexés. Des modifications restent possibles, notamment du point de vue de la constitution des divers éléments sans sortir pour autant du domaine de protection de l'invention comme définie dans les revendications ci-annexées.

## Revendications

1. Dispositif de maintien et de déplacement contrôlé en translation, dans sa direction longitudinale, d'un corps allongé en forme de tige, du type aiguille, et en particulier d'une aiguille pour procédure médicale percutanée, dispositif destiné à être monté sur un support réglable de manière contrôlée en position et en orientation, tel que par exemple une plateforme ou un bras d'un dispositif robotique de positionnement et d'orientation, ce dispositif (1) comprenant essentiellement, d'une part, un premier organe (5) de préhension du corps allongé (2), déplaçable par rapport au support (3) de manière commandée et/ou contrôlée dans la direction de translation (Z) avec une course maximale prédéterminée, et, d'autre part, un second organe (6) fixe par rapport au support (3) et apte à guider le corps allongé (2) lorsque ce dernier est déplacé par le premier organe mobile (5) de préhension et, le cas échéant, à maintenir ledit corps allongé (2) en position lorsque ce dernier n'est pas en prise avec ledit premier organe (5), les deux organes (5 et 6) étant alignés dans la direction de translation (Z) et le premier organe (5) pouvant être déplacé de manière alternative entre une position proximale du second organe (6) et une position distante de ce dernier, l'organe de préhension (5) mobile comprenant des moyens (7) de serrage par pincement et avec blocage du corps allongé (2) pouvant être déplacé en rotation avec le corps allongé (2) autour de l'axe de translation (Z), dispositif (1) **caractérisé en ce que** l'organe de préhension (5) consiste en un mandrin recevant le corps allongé du type aiguille (2) de manière traversante, **en ce que** les mouvements de serrage et de desserrage du mandrin (5) sont commandés par un organe de manoeuvre rotatif (9) et **en ce que** les moyens de serrage (7) sous la forme de mors sont montés, avec faculté de coulissement guidé, dans un corps (10) avec un passage tubulaire (10") destiné à recevoir de manière entourante et traversante le corps allongé (2), le déplacement desdits moyens de serrage (7) étant généré par un mouvement de rotation dudit organe de manoeuvre (9).

2. Dispositif selon la revendication 1, **caractérisé en ce que** l'organe de manoeuvre rotatif (9) se présente sous la forme d'une couronne de manoeuvre dentée ou crantée, intégrée audit mandrin (5) et **en ce que** le déplacement des mors (7) est généré par un mouvement de rotation de ladite couronne de manoeuvre (9) entourant le corps (10), le mandrin (5) présentant avantageusement, à l'état ouvert, un degré d'ouverture suffisant pour garantir un relâchement total du corps (2), avec seulement un maintien latéral avec battement.

3. Dispositif selon la revendication 1 ou 2, **caractérisé en ce que** le mandrin (5) formant l'organe de préhension mobile est monté sur ou dans un chariot (8) pouvant être déplacé par entraînement motorisé dans les deux sens opposés de la direction de translation (Z) dudit corps allongé (2), en étant guidé dans une structure support (15) allongée apte à être assemblée rigidement avec la plateforme ou le bras (3) d'un dispositif robotique (4) de positionnement et d'orientation, ladite structure support (15) étant avantageusement conformée pour autoriser un accès latéral au corps (2) engagé dans ladite structure support (15) et éventuellement une mise en place et une extraction en biais latéralement ou radialement du corps (2), le cas échéant avec le mandrin (5).

4. Dispositif selon la revendication 1 ou 2, **caractérisé en ce que** le mandrin (5) formant l'organe de préhension mobile est reçu directement dans un corps support (15) allongé avec faculté de coulissement guidé dans la direction de translation (Z) du corps allongé (2) et avec faculté de rotation autour dudit axe (2), ledit corps support (15) formant glissière pour ledit mandrin mobile (5), dont le déplacement résulte préférentiellement directement de l'action manuelle d'un opérateur, ladite structure support (15) étant avantageusement conformée pour autoriser un accès latéral au corps (2) engagé dans ladite structure support (15) et éventuellement une mise en place et une extraction en biais latéralement ou radialement du corps (2).

5. Dispositif selon l'une quelconque des revendications 3 et 4, **caractérisé en ce que** le second organe fixe (6) est situé au niveau de l'extrémité du corps support (15) de structure allongée, qui est distale par rapport à la plateforme ou au bras (3) portant ledit dispositif (1), en étant un corps (10') rapporté sur ce corps support (15) ou formé par une partie de ce dernier.

6. Dispositif selon l'une quelconque des revendications 1 à 5, **caractérisé en ce que** le second organe fixe (6) consiste en un palier de guidage ajusté du corps allongé (2), la friction entre ce dernier et ledit palier étant au moins suffisante pour assurer un maintien dudit corps allongé (2) à l'encontre de la pesanteur.

7. Dispositif selon l'une quelconque des revendications 1 à 5, **caractérisé en ce que** le second organe fixe (6) consiste en un orifice ou canal de guidage avec jeu du corps allongé (2), destiné à limiter le battement radial ou l'inclinaison angulaire du corps allongé (2) par rapport à la direction de translation (Z) et à maintenir ledit corps allongé (2) sensiblement dans une disposition coaxiale avec ladite direction (Z), ce en coopération avec le mandrin (5) formant organe mobile de préhension et d'entraînement.

8. Dispositif selon la revendication 2, **caractérisé en ce que** le second organe fixe (6) réalise une fonction de préhension contrôlée du corps allongé (2) et consiste en un mandrin recevant le corps allongé (2) de manière traversante, ce second mandrin (6) présentant une structure, un mode de fonctionnement et des éléments constitutifs identiques à ceux du premier mandrin (5), à savoir notamment une couronne de manoeuvre (9'), des mors (7') et un corps (10') avec un passage tubulaire (10").

9. Dispositif selon l'une au moins des revendications 2 et 8, **caractérisé en ce que** le ou chaque mandrin (5, 6) consiste essentiellement en un corps (10, 10') à passage traversant tubulaire (10"), ouvert latéralement pour permettre le passage d'au moins deux, préférentiellement trois, mors (7, 7') et pourvu de moyens de guidage (11, 11') desdits mors (7, 7') dans une direction radiale par rapport au passage tubulaire du corps (10, 10') concerné, **en ce que** lesdits mors (7, 7') sont répartis uniformément autour dudit passage (10") et peuvent être déplacés entre une position dite de serrage dans laquelle ils s'étendent partiellement dans le passage (10") correspondant et une position dans laquelle ils libèrent totalement ledit passage (10"), et **en ce que** lesdits mors (7, 7') comportent des segments de nervure / rainure (12) aptes à venir en engagement et à circuler dans une rainure / nervure (13) en portion de spirale ménagée dans une face latérale d'une couronne de manoeuvre dentée (9, 9') montée avec faculté de rotation sur une portion à forme externe tubulaire (10''') du corps (10, 10') du mandrin concerné (5, 6), une rotation de ladite couronne (9 ou 9') entraînant un coulissement radial simultané et identique des mors (7 ou 7').

10. Dispositif selon la revendication 9, **caractérisé en ce que** le ou chaque mandrin (5, 6) est monté avec faculté de rotation autour de l'axe longitudinal (Z') du corps allongé (2) qui le traverse, le cas échéant, ensemble avec ce dernier, et **en ce qu'**à chaque mandrin (5, 6) est associé un mécanisme à cliquet (14, 14') bloquant le corps (10, 10') dudit mandrin (5, 6) concerné contre une rotation dans le sens du desserrage et autorisant une rotation progressive dudit mandrin (5, 6), dans le sens de serrage, lorsque ce dernier est intimement serré autour du corps allongé (2) et qu'un couple de serrage supplémentaire, présentant une intensité supérieure à une valeur déterminée définie par le mécanisme à cliquet (14, 14') concerné, est appliqué au niveau de la couronne de manoeuvre dentée (9, 9') correspondante.

11. Dispositif selon l'une quelconque des revendications 1 à 8, **caractérisé en ce que** le premier organe mobile (5) et le second organe fixe (6) sont montés, préférentiellement de manière amovible, sur une structure support (15) apte à être assemblée rigidement avec la plateforme ou le bras (3) d'un dispositif robotique (4) de positionnement et d'orientation, la structure support (15) sous la forme d'un châssis présentant une extension donnée dans la direction de déplacement en translation (Z) du corps allongé (2), comportant un site de montage (16) pour le second organe fixe (6), positionné de manière extrémale dans le sens correspondant à un déplacement positif ou d'enfoncement dudit corps allongé (2) dans la direction de translation (Z), et étant munie de moyens (17, 18) de guidage et de déplacement contrôlé du premier organe mobile (5) qui est monté, au niveau d'un site de montage (19) correspondant adapté, sur ou dans un chariot (8) coopérant avec ces moyens (17, 18).

**12.** Dispositif selon la revendication 11, **caractérisé en ce que** les moyens de guidage et de déplacement contrôlé du premier organe mobile (6) comprennent, d'une part, un arbre fileté (17) formant vis mère et, d'autre part, au moins un rail ou une tige de guidage (18), et **en ce que** le chariot (8) intègre une perforation filetée (8') formant écrou fils et au moins un palier ou patin de guidage (8"), traversant ou non, qui coopère, de manière coulissante, avec le ou chaque rail ou tige de guidage (18).

**13.** Dispositif selon la revendication 11 ou 12, **caractérisé en ce que** les premier et second organes (5 et 6), se présentant sous forme de mandrins, sont reçus de manière amovible et avec faculté de rotation dans leurs sites de montage respectifs (16 et 19) sensiblement en forme de U, **en ce que** les corps (10, 10') des mandrins (5, 6), de forme générale cylindrique circulaire, présentent une rainure circonférentielle externe (20, 20') et les sites de montage (16 et 19) présentent chacun une nervure (21, 21') apte à coopérer avec la rainure (20, 20') du corps (10, 10') de mandrin (5, 6) correspondant, sur une partie de leur extension circonférentielle, en vue de la rétention dudit corps (10, 10') et **en ce que** lesdits sites de montage (16, 19) sont ouverts latéralement pour permettre la mise en place et l'enlèvement desdits mandrins (5, 6), ces derniers étant verrouillés à l'état monté dans leurs sites respectifs (16, 19) par des pièces de blocage mobiles (22, 22'), préférentiellement sollicitées élastiquement en position de verrouillage desdits mandrins (5, 6), ces pièces de blocage (22, 22') venant en prise dans les rainures externes (20, 20') des corps (10, 10') des mandrins respectivement concernés (5, 6), dans la continuité des nervures (21,21') et en fermant les ouvertures latérales (23, 23') des sites de montage respectifs (16, 19).

**14.** Dispositif selon la revendication 13, **caractérisé en ce que** les premier et second organes (5 et 6) sont montés dans leurs sites de montage (19 et 16) respectif à l'encontre d'une sollicitation élastique, cette dernière éjectant lesdits organes (5 et 6) hors desdits sites (19 et 16) lorsque les pièces de blocage (22, 22'), préférentiellement commandées de manière simultanée, libèrent les ouvertures latérales (23 et 23') correspondantes.

**15.** Dispositif selon l'une quelconque des revendications 3 et 5 à 14, pour autant que ces dernières dépendent de la revendication 3, **caractérisé en ce que** le chariot (8) portant organe de préhension (5) est relié de manière amovible audit dispositif (1), en pouvant notamment être déplacé dans une position de coulissement extrême au niveau de laquelle il se désolidarise dudit dispositif (1), le chariot (8) recevant avantageusement le mandrin (5), de manière amovible et avec faculté de rotation, dans un logement annulaire de forme adaptée, comportant une ouverture d'introduction obturable par un couvercle (62) amovible. .

**16.** Dispositif selon la revendication 8 et l'une quelconque des revendications 9 à 15 pour autant qu'elles se rattachent à la revendication 8, **caractérisé en ce que** l'entraînement de la couronne de manoeuvre (9, 9') de chaque organe en forme de mandrin (5, 6) est réalisé par l'intermédiaire d'un pignon (24, 25) engrenant avec ladite couronne (9, 9') et solidaire en rotation avec une tige ou un arbre (24', 25') entraîné(s) en rotation par un actionneur (24", 25") respectif correspondant, éventuellement communs, **en ce que** le pignon (25) engrenant avec la couronne (9') du mandrin fixe (6) est solidarisé rigidement au niveau de l'extrémité d'un arbre d'entraînement (25') correspondant et **en ce que** le pignon (24) engrenant avec la couronne (9) du mandrin mobile (5) est solidaire en rotation et libre en translation par rapport à un arbre d'entraînement (24') correspondant.

**17.** Dispositif selon la revendication 13, **caractérisé en ce qu'**il comporte une tige ou tringle de commande (26) pivotante ou coulissante, reliée en entraînement avec les tiges de blocage (22, 22') en vue de leur déplacement hors de leur position de verrouillage à l'encontre de forces élastiques de sollicitation, par exemple fournies par des ressorts de compression (27, 27'), cette tige de commande (26) étant solidaire en entraînement d'un mécanisme d'actionnement (26'), en particulier un mécanisme de sécurité provoquant un déverrouillage par défaut.

**18.** Dispositif selon les revendications 16 et 17, **caractérisé en ce que** les arbres (24' et 25'), ainsi que la tige de commande (26), présentent des extrémités libres, du côté des actionneurs (24", 25"), respectivement du mécanisme (26'), effilées ou en pointe, notamment aptes à percer la paroi d'un conditionnement stérile.

**19.** Dispositif selon l'une quelconque des revendications 16 à 18, pour autant qu'elles dépendent de la revendication 11, **caractérisé en ce que** le châssis formant structure support (15) comporte un plateau inférieur (15') renfermant le site de montage extrémal (16) pour le second organe (6) et un plateau supérieur (15") de fixation à la plateforme (3) d'un dispositif robotique (4) de fonctionnement et d'orientation, ledit plateau supérieur (15") étant pourvu de moyens d'assemblage rapide (28), tels que par exemple des loquets, aptes à venir en prise avec ladite plateforme (3) ou avec une pièce solidaire de cette dernière à l'état monté dudit châssis (15), et **en ce que** les moyens (17, 18) de guidage et de déplacement contrôlé, ainsi que le cas échéant les arbres d'entraînement (24', 25') et la tige de commande (26), s'étendent et sont montés entre ces deux plateaux (15' et 15") dont l'espacement mutuel détermine sensiblement la course du premier organe mobile (5).

20. Dispositif selon l'une quelconque des revendications 1 à 19, **caractérisé en ce qu'**il comprend des capteurs d'effort (29) mesurant l'intensité de la poussée exercée sur le corps allongé (2) dans la direction de translation (Z), et le cas échéant du couple ou de certaines composantes au moins du couple exercé sur ce corps allongé (2).

21. Dispositif selon la revendications 20 pour autant qu'elle se rattache à la revendication 19, **caractérisé en ce que** les capteurs d'effort (29) sont disposés entre un plateau supérieur (15") de la structure support (15) et une plateforme (3) du dispositif robotique (4), en étant préférentiellement montés sur un plateau (30) intercalaire pris en sandwich entre ledit plateau supérieur (15") et ladite plateforme (3).

22. Dispositif selon la revendication 20 ou la revendication 21, **caractérisé en ce que** les capteurs d'effort (29) consistent en des capteurs unidirectionnel orientés dans la direction de translation (Z), sont au nombre de trois et sont disposés dans un plan orthogonal à ladite direction (2) et positionnés de manière symétrique sur un cercle de rayon (r) centré sur l'axe longitudinal (Z') du corps allongé (2), cet arrangement de trois capteurs (29) autorisant ainsi la détermination de deux composantes du couple de réaction exercé sur le corps allongé (2).

23. Dispositif selon les revendications 16 et 17, **caractérisé en ce que** les actionneurs (24", 25") entraînant les arbres (24', 25') sont regroupés sur un plateau support (31) rapporté sur la plateforme (3) du dispositif robotique (4) à l'opposé de la structure support (15) portant les premier et second organes (5 et 6), ce plateau support (31) portant, le cas échéant, également le mécanisme d'actionnement (26') de la tige de commande (26) apte à déplacer les pièces de blocage (22, 22') hors de leur position verrouillée.

24. Dispositif selon la revendication 23, **caractérisé en ce que** les actionneurs (24", 25") des arbres (24', 25') se présentent sous la forme d'un actionneur double intégrant un unique moteur (35) à couple élevé et à faible vitesse de rotation, par exemple du type piézoélectrique, et un arrangement (36) d'engrenages de transmission transformant le mouvement de rotation de sortie dudit moteur en deux mouvements de rotation synchrones et de sens opposés, fournis aux deux arbres (24' et 25').

25. Dispositif selon les revendications 12 et 33, **caractérisé en ce que** le plateau support (31) porte également un actionneur (17') pour l'arbre fileté (17) déplaçant le chariot (8), ce par exemple sous la forme d'un moteur piézoélectrique associé à un mécanisme d'engrenage.

26. Dispositif selon la revendication 2, **caractérisé en ce que** les mors (7) formant les moyens de serrage de mandrin (5) sont montés guidés dans le passage traversant tubulaire (10") du corps (10), lesdits mors (7) étant pourvus de moyens d'entraînement (39) sollicités, directement ou indirectement, par la couronne de manoeuvre (9) entourante, de manière à être déplacés en translation selon (Z ou Z') lors de la rotation de cette couronne (9), en générant un mouvement simultané de rapprochement ou d'éloignement mutuel desdits mors (7) autour de l'axe médian dudit passage (10") ou d'un corps allongé (2) monté dans ledit mandrin (5).

27. Dispositif selon la revendication 26, **caractérisé en ce que** les moyens de serrage (7) consistent en deux mors opposés et en regard, avec des mâchoires (7") mutuellement décalées de manière à s'interpénétrer lors de leur mouvement de rapprochement en vue du serrage, **en ce que** le passage tubulaire (10") présente deux faces opposées planes (37) formant surfaces de guidage plan pour lesdits mors (7), ces derniers étant également pourvus de portions de surfaces de guidage latérales (38) adaptées pour glisser en appui sur lesdites faces (37) et **en ce que** chacun desdits mors (7) est guidé de manière supplémentaire durant leurs mouvements de rapprochement et d'écartement mutuel pour effectuer simultanément un déplacement dans la direction (Z ou Z').

28. Dispositif selon 1a revendication 27, **caractérisé en ce que** chacun desdits mors (7) est pourvu d'au moins deux tiges d'entraînement et de guidage (39") saillantes latéralement par rapport à ses portions de surfaces de guidage (38) et formant chacune une paire d'ergots ou de doigts (39) opposés, ces derniers étant, d'une part, montés coulissant dans des portions de rainures (40) ménagées dans les portions de paroi du corps (10) définissant les faces opposées planes (37), coïncidentes deux par deux en cas de superposition des deux faces opposées planes (37) et situées par paires coïncidentes dans des plans ou des surfaces non planes inclinées par rapport à la direction longitudinale (Z') d'un corps allongé (2) monté de manière serrée dans le mandrin (5) et, d'autre part, au moins pour une paire de doigts ou d'ergots (39) opposés de chaque mors (7), en relation d'entraînement, directe ou indirecte, avec la couronne de manoeuvre (9) au niveau de leurs extrémités (39') dépassant des portions de rainures (40) qu'ils traversent, en vue de leur déplacement dans la direction longitudinale (Z') lors d'un mouvement de rotation de ladite couronne de manoeuvre (9).

**29.** Dispositif selon l'une quelconque des revendications 27 et 28, **caractérisé en ce que** le rapport entre l'ouverture interne maximale du mandrin (5), pour un écartement maximal des mors (7), et le diamètre externe de la couronne (9) est supérieur à 0,2, préférentiellement d'au moins 0,3.

**30.** Dispositif selon la revendication 28, **caractérisé en ce que** le corps (10) présente une forme extérieure cylindrique circulaire, ajustée en diamètre au diamètre intérieur de la couronne de manoeuvre (9), les extrémités (39') des doigts ou ergots (39) qui dépassent des portions de rainure (40) au niveau de la face externe du corps (10) venant en engagement glissant guidé dans des rainures (41) ménagées au niveau de la face interne de la couronne de manoeuvre (9) et formées par des portions de courbes engendrées chacune par l'intersection entre une surface réglée et la surface cylindrique interne de ladite couronne (9), le corps (10) étant bloqué en translation et libre en rotation dans cette couronne (9) entourante.

**31.** Dispositif selon la revendication 28, **caractérisé en ce que** le corps (10) présente une forme extérieure carrée ou rectangulaire et est monté dans la couronne de manoeuvre (9) avec interposition d'une pièce intermédiaire (42) présentant une forme extérieure cylindrique circulaire, ajustée en diamètre au diamètre intérieur de la couronne (9) et ayant une forme intérieure complémentaire de la forme extérieure du corps (10), cette pièce intermédiaire (42) étant pourvue, d'une part, de rainures (43) coïncidentes par paires, disposées par paires dans des plans perpendiculaires à l'axe médian du passage tubulaire (10") et recevant, avec un engagement coulissant guidé, les extrémités (39') des doigts ou ergots (39) qui dépassent des portions de rainures (40) au niveau de la face externe du corps (10) et, d'autre part, d'au moins deux ergots (44) saillants au niveau de sa face extérieure et venant en engagement glissant guidé dans des rainures (41) sensiblement en forme de portions d'hélices et ménagées au niveau de la face interne de la couronne de manoeuvre (9), le corps (10) étant bloqué en translation et libre en rotation dans cette couronne (9) entourante.

**32.** Dispositif selon l'une quelconque des revendications 26 à 31, **caractérisé en ce que** la couronne de manoeuvre (9) présente sur sa face extérieure périphérique une denture inclinée (45), adaptée à un engagement fonctionnel avec une vis sans fin (46) en vue de son entraînement en rotation, un entraînement de ladite couronne (9) au-delà d'un degré de serrage déterminé du corps allongé (2) par les mors (7), provoquant une rotation du mandrin (5) et du corps (2) serré.

**33.** Dispositif selon les revendications 2 et 3 ou l'une quelconque des revendications 26 à 30 pour autant qu'elle se rattache aux revendications 2 et 3, **caractérisé en ce que** le mandrin (5) est assujetti, de manière amovible et avec faculté de rotation, dans un chariot (8) monté coulissant dans la direction de translation (Z) dans la structure support allongé (15) munie d'au moins une glissière (47) adaptée, ladite structure (15) étant pourvue d'un plateau supérieure (15") pour sa fixation sur la plateforme (3) du dispositif robotique (4) et d'un plateau inférieur (15') comportant un orifice ou canal de guidage avec jeu et de centrage, formant le second organe fixe (6), et **en ce qu'**un ensemble motorisé (48) de déplacement contrôlé du chariot (8) et un ensemble motorisé (49) d'actionnement de la couronne de manoeuvre (9) sont associés à l'ensemble chariot (8) / corps support (15), en étant montés soit sur la plateforme (3), soit sur ledit corps support (15).

**34.** Dispositif selon la revendication 33, **caractérisé en ce que** l'ensemble motorisé (48) consiste en une vis mère (51) entraînée en rotation par un bloc-moteur (52) associé éventuellement à un codeur de position, et entraînant en translation un écrou fils (53), ledit écrou fils (53) étant relié rigidement à un chariot ou une paire de chariots de guidage intermédiaire(s) (54) coulissant sur un rail (55) parallèle à la direction de translation (Z) et à la glissière (47), **en ce que** ledit ou lesdits chariot(s) (54) est (sont) également relié(s) rigidement à un site (56') d'accouplement amovible d'une barre de liaison (56) solidaire du chariot (8), **en ce que** les points de fixation et de liaison dudit écrou fils (53) et dudit site d'accouplement (56') avec le ou l'un des chariots (54) sont reliés entre eux avec interposition d'un capteur d'effort (54') apte à mesurer l'intensité des actions mécaniques dans la direction (Z), et **en ce que** ledit dispositif comporte des fins de course (57) opposés, mutuellement décalés dans la direction de translation (Z), et délimitant la course maximale dudit ou desdits chariot(s) intermédiaire(s) (54) et donc du chariot (8), ledit ensemble motorisé (48), à l'exception des fins de course (57), étant éventuellement monté dans un carter (60), formé d'un seul tenant avec la plateforme (3) ou reçu, de manière indexée et avec blocage en position, dans un site de réception (61) solidaire de la plateforme (3), en formant ainsi un module autonome amovible.

**35.** Dispositif selon la revendication 33, **caractérisé en ce que** l'ensemble motorisé (49) consiste en un bloc-moteur (58) entraînant, par l'intermédiaire d'un arbre de transmission flexible (59), une vis sans fin (46) intégrée au chariot (8) et en engagement d'entraînement avec la couronne de manœuvre (9) périphérique du mandrin (5) monté dans ledit chariot (8), ledit bloc-moteur (58) étant monté dans un carter formé d'un seul tenant avec la plateforme (3) ou reçu de manière indexée et avec blocage en position dans un site de réception de ladite plateforme (3).

**36.** Dispositif selon l'une quelconque des revendications 1 à 35, **caractérisé en ce que** le corps allongé (2) consiste en une aiguille pour la réalisation de procédures médicales percutanées.

**37.** Dispositif selon les revendications 13, 19, 21 et 33, **caractérisé en ce que** la plateforme (3) du dispositif robotique (4) présente une découpe ouverte (3'), de même qu'éventuellement le plateau supérieur (15") de la structure support (15), le plateau intercalaire (30) portant les capteurs d'effort (29) et le plateau support (31) des actionneurs (17', 24', 25'), l'ouverture de cette ou ces découpe(s) étant orientée dans la même direction que les ouvertures (23, 23') des sites de montage (16 et 19) des premier et second organes (5 et 6) et/ou qu'un dégagement latéral de la structure (15), de manière à permettre une mise en place et/ou un enlèvement latéral(e) de ladite aiguille (2).

**38.** Système d'assistance aux procédures médicales percutanées guidées par imagerie médicale, comprenant un dispositif robotique de positionnement et d'orientation d'une plateforme, un dispositif d'insertion et de rétraction contrôlées d'aiguille et un dispositif de pilotage desdits dispositifs en fonction de paramètres préprogrammés et de résultats de mesures instantanées, telles que des mesures extraites d'images médicales peropératoires, des mesures de sollicitations dans la direction axiale de l'aiguille et/ou des sollicitations de couple au niveau de ladite aiguille, ou encore des indications ou mesures de position fournies par le dispositif robotique et/ou le dispositif d'insertion précités, système **caractérisé en ce que** le dispositif d'insertion et de rétraction d'aiguille consiste en un dispositif (1) selon l'une quelconque des revendications 1 à 37.

**39.** Système selon la revendication 38, **caractérisé en ce que** le dispositif (1) d'insertion et d'extraction d'aiguille (2) présente une structure modulaire étagée, avec un premier module (32) renfermant les premier et second organes (5 et 6) et les moyens de commande mécaniques de ces derniers, un second module (33) renfermant des moyens capteurs, et un troisième module (34) renfermant les moyens d'actionnement des moyens de commande mécaniques précités, les second et troisième modules (33 et 34) étant conditionnés dans un emballage stérile, ensemble avec une partie attenante au moins du dispositif robotique (4) de positionnement et d'orientation, et le premier module (32) étant réalisé, partiellement ou totalement, en tant que module du type consommable ou à usage unique.

**40.** Système selon la revendication 38, **caractérisé en ce que** le corps support (15), le cas échéant avec le chariot (8) et éventuellement le flexible (59), forme(nt) un module du type consommable ou à usage unique, la partie complémentaire du système étant conditionnée dans un emballage stérile.

## Claims

**1.** Device for holding and for the controlled translational movement, in its longitudinal direction, of a rod-shaped elongated body, in particular of the needle type, and in particular a needle for a percutaneous medical procedure, said device being designed to be mounted on an adjustable support in a manner that is controlled in position and in orientation, such as for example a platform or an arm of a robotic device for positioning and orientation, wherein said device (1) essentially comprises on the one hand a first part (5) for gripping the elongated body (2), movable relative to the support (3) in a regulated and/or controlled manner in translational direction (Z) with a predetermined maximum course, and on the other hand a second part (6) that is stationary relative to the support (3) and able to guide the elongated body (2) when the latter is moved by the first movable gripping part (5) and if necessary to keep said elongated body (2) in position when the latter is not engaged with said first part (5), wherein the two parts (5 and 6) are aligned in translational direction (Z) and wherein the first part (5) can be moved alternatively between a proximal position of the second part (6) and a distant position of the latter, wherein the movable gripping part (5) comprises clamping means (7) where the elongated body (2) is locked and can be moved in rotation with the elongated body (2) around the translational axis (Z), said device (1) being **characterised in that** the gripping part (5) consists of a mandrel receiving the elongated body of the needle type (2) in a traversing manner, **in that** the tightening and loosening movements of the mandrel (5) are controlled by a rotary manoeuvring part (9), and **in that** the clamping means (7) in the form of a jaws are mounted with the capability of guided sliding, in a body (10) with a tubular passage (10") that is designed to hold the elongated body (2) in a surrounding and traversing manner, the movement of said clamping means (7) being generated by a rotational movement of said manoeuvring part (9).

2. Device according to claim 1, **characterised in that** the rotational manoeuvring part (9) is in the form of a toothed or crowned ring, integral with said mandrel (5) and **in that** the movement of the jaws (7) is generated by a rotational movement of said manoeuvring ring (9) surrounding the body (10): the mandrel (5) preferably comprising, in the open state, a degree of opening sufficient to ensure the total release of the body (2) with only lateral support with wobbling.

3. Device according to claim 1 or 2, **characterised in that** the mandrel (5) that forms the moving gripping part is mounted on or in a cart (8) that can be moved by motorised driving in two opposite directions from the translational direction (Z) of said elongated body (2) being guided in an elongated support structure (15) that is able to be assembled rigidly with the platform or the arm (3) of a robotic device (4) for positioning and orientation, wherein said support structure (15) is advantageously shaped so as to allow lateral access to the body (2) that is engaged in said support structure (15) and optionally an installation and an extraction at a lateral or radial slant from the body (2), if necessary with the mandrel (5).

4. Device according to claim 1 or 2, **characterised in that** the mandrel (5) that forms the moving gripping part is accommodated directly in a support body (15) that is elongated with the capability of guided sliding in the translational direction (Z) of the elongated body (2) and with the capability of rotation around said axis (2), wherein said support body (15) forms a slide for said moving mandrel (5), the movement of which preferably results directly from the manual action of an operator, wherein said support structure (15) is advantageously shaped so as to allow lateral access to the body (2) that is engaged in said support structure (15) and optionally an installation and an extraction at a lateral or radial slant from the body (2).

5. Device according to any one of claims 3 and 4, **characterised in that** the second stationary part (6) is located at the end of the support body (15) with an elongated structure, which is distal relative to the platform or to the arm (3) that holds said device (1), by being a body (10') that is connected to this support body (15) or formed by a portion of the latter.

6. Device according to any one of claims 1 to 5, **characterised in that** the second stationary part (6) consists of an adjusted guide bearing of the elongated body (2), wherein the friction between the latter and said bearing is at least enough to ensure that said elongated body (2) is held against gravity.

7. Device according to any one of claims 1 to 5, **characterised in that** the second stationary part (6) consists of an opening or a guide channel with play of the elongated body (2), designed to limit the radial wobbling or angular inclination of the elongated body (2) relative to the translational direction (Z) and to hold said elongated body (2) essentially in a coaxial arrangement with said direction (Z), in cooperation with the mandrel (5) that forms a moving gripping and driving part.

8. Device according to claim 2, **characterised in that** the second stationary part (6) performs a controlled gripping function of the elongated body (2) and consists of a mandrel through which the elongated body (2) extends, wherein this second mandrel (6) has a structure, a mode of operation and constituent parts that are identical to those of the first mandrel (5), namely
in particular a manoeuvring ring (9'), jaws (7') and a body (10') with a tubular passage (10").

9. Device according to any one of claims 2 to 8, **characterised in that** the mandrel (5, 6) or each mandrel (5, 6) essentially consists of a body (10, 10') with a tubular through passage (10"), opening laterally for allowing the passage of at least two, preferably three, jaws (7, 7') and is equipped with guide means (11, 11') for said jaws (7, 7') in radial direction relative to the tubular passage of the body (10, 10') in question, wherein said jaws (7, 7') are distributed uniformly around said passage (10") and can be moved between a so-called clamping position in which they extend partially into the corresponding passage (10") and a position in which they totally release said passage (10"), and wherein said jaws (7, 7') comprise rib/groove segments (12) that can be engaged and that can circulate in a groove/rib (13) in a spiral portion that is formed in a lateral face of a toothed manoeuvring ring gear (9, 9') that is mounted with the capability of rotation on a portion with a tubular external shape (10''') of the body (10, 10') of the mandrel in question (5, 6), wherein a rotation of said ring (9 or 9') entrains a simultaneous or identical radial slide of the jaws (7 or 7').

10. Device according to claim 9, **characterised in that** the mandrel (5, 6) or each mandrel (5, 6) is mounted with the capability of rotation around the longitudinal axis (Z') of the elongated body (2) that passes through it, if necessary together with the latter, and wherein each mandrel (5, 6) is associated with a pawl mechanism (14, 14') that locks the body (10, 10') of said mandrel (5, 6) in question against rotation in the direction of loosening and allows a gradual rotation of said mandrel (5, 6), in the direction of clamping when the latter is thoroughly tightened around the elongated body (2), and wherein an additional tightening torque, having an intensity that is greater than a determined value defined by the pawl mechanism (14, 14') in question is applied at the corresponding toothed manoeuvring ring (9, 9').

11. Device according to any one of claims 1 to 8, **characterised in that** the first moving part (5) and the second stationary part (6) are mounted, preferably in a removable manner, on a support structure (15) that can be assembled rigidly with the platform or the arm (3) of a robotic device (4) for positioning and orientation, wherein the support structure (15) in the form of a chassis has a given extension in the translational movement direction (Z) of the elongated body (2), comprising a mounting site (16) for the second stationary part (6), positioned at the extreme end in the direction corresponding to a positive movement or embedding of said elongated body (2) in the translational direction (Z), and being equipped with means (17, 18) for guiding and for controlled movement of the first moving part (5) that is mounted, at a suitable corresponding mounting site (19), on or in a cart (8) that cooperates with these means (17, 18).

12. Device according to claim 11, **characterised in that** the means for guiding and controlled movement of the first moving part (6) comprise, on the one hand, a threaded shaft (17) that forms a mother screw, and, on the other hand, at least one guide rail or rod (18), and **in that** the cart (8) integrates a threaded perforation (8') that forms a wire nut and at least one bearing or guide shoe (8") that may or may not pass through and that works, in a sliding manner, with the guide rail or rod (18) or each guide rail or rod (18).

13. Device according to claim 11 or 12, **characterised in that** the first and second parts (5 and 6) in the form of mandrels are accommodated in a removable manner and with the capability of rotation in their respective essentially U-shaped mounting sites (16 and 19), **in that** the bodies (10, 10') of the mandrels (5, 6) that are generally circular-cylindrical in shape have an external circumferential groove (20, 20'), and the mounting sites (16 and 19) each have a rib (21, 21') that can cooperate with the groove (20, 20') of the corresponding body (10, 10') of the mandrel (5, 6), over a portion of their circumferential extension, for the purpose of retaining said body (10, 10'), and **in that** said mounting sites (16, 19) are open laterally to allow the installation and removal of said mandrels (5, 6), wherein the latter are locked in the mounted state in their respective sites (16, 19) by moving locking pieces (22, 22'), preferably stressed elastically in the locking position of said mandrels (5, 6), wherein said locking pieces (22, 22') become engaged in the external grooves (20, 20') of the bodies (10, 10') of the respective mandrels (5, 6) in the continuity of the ribs (21, 21') and by closing the lateral openings (23, 23') of respective mounting sites (16, 19).

14. Device according to claim 13, **characterised in that** the first and second parts (5 and 6) are mounted in their mounting sites (19 and 16) respectively against elastic stress, wherein the latter ejects said parts (5 and 6) beyond said sites (19 and 16) when the locking pieces (22, 22'), preferably controlled simultaneously, release the corresponding lateral openings (23 and 23').

15. Device according to any one of claims 3 and 5, insofar as the latter are dependent on claim 3, **characterised in that** the cart (8) that holds the gripping part (5) is connected in a removable manner to said device (1) by being able to be moved in particular into an end sliding position at the level of which it disengages from said device (1), wherein the cart (8) preferably receives the mandrel (5), in a removable manner and with the ability to rotate, in an annular housing with an adjusted shape, comprising an introduction opening which can be closed by a removable lid (62).

16. Device according to claim 8 and any one of claims 9 to 15 insofar as they relate to claim 8, **characterised in that** the driving of the manoeuvring ring (9, 9') of each part in the form of a mandrel (5, 6) is carried out by means of a pinion (24, 25) that engages with said ring (9, 9') and is integral in rotation with a rod or a shaft (24', 25') that is driven in rotation by a corresponding, optionally joint, actuator (24", 25"), wherein the pinion (25) that engages with the ring (9') of the stationary mandrel (6) is made integral rigidly at the end of a corresponding drive shaft (25'), and wherein the pinion (24) that engages with the ring (9) of the moving mandrel (5) is integral in rotation and free in translation relative to a corresponding drive shaft (24').

17. Device according to claim 13, **characterised in that** it comprises a pivoting or sliding control rod or pole (26) that is drive-connected with the locking rods (22, 22') for the purpose of their displacement beyond their locking position against elastic stressing forces, for example provided by compression springs (27, 27'), wherein this control rod (26) is integral in driving an actuating mechanism (26'), in particular a safety mechanism that causes unlocking by default.

18. Device according to claims 16 and 17, **characterised in that** the shafts (24' and 25') as well as the control rod (26) have free ends, on the side of the actuators (24", 25"), respectively of the mechanism (26'), tapered or pointed, in particular able to pierce the wall of a sterile package.

19. Device according to any one of claims 16 to 17, insofar as they depend on claim 11, **characterised in that** the chassis that forms a support structure (15) comprises a lower plate (15') that contains the mounting site at the extreme end (16) for the second part (6) and an upper plate (15") for attachment to the platform (3) of a robotic device (4) for operation and orientation, wherein said upper plate (15") is equipped with quick assembly means (28), such as, for example, latches, able to engage with said platform (3) or with a piece that is integral with the latter in the mounted state of said chassis (15), and wherein the means (17, 18) for guiding and controlled movement, as well as, if necessary, the drive shafts (24', 25') and the control rod (26), extend and are mounted between these two plates (15' and 15"), the mutual spacing of which essentially determines the travel of the first moving part (5).

20. Device according to any one of claims 1 to 19, **characterised in that** it comprises force sensors (29) that measure the intensity of the thrust exerted on the elongated body (2) in the translational direction (Z), and, if necessary, the torque or certain components at least of the torque exerted on this elongated body (2).

21. Device according to claim 20, insofar as it relates to claim 19, **characterised in that** the force sensors (29) are arranged between the upper plate (15") of the support structure (15) and the platform (3) of the robotic device (4) by preferably being mounted on a separator (30) that is sandwiched between said upper plate (15") and said platform (3).

22. Device according to claim 20 or claim 21, **characterised in that** the force sensors (29) consist of unidirectional sensors that are oriented in the translational direction (Z), are three in number and are arranged in a plane that is orthogonal to said direction (2) and positioned symmetrically on a circle with radius (r) that is centred on the longitudinal axis (Z') of the elongated body (2), wherein this arrangement of three sensors (29) thus allows the determination of two components of the reaction torque exerted on the elongated body (2).

23. Device according to claims 16 and 17, **characterised in that** the actuators (24", 25") that drive the shafts (24', 25') are grouped on a support plate (31) that is connected to the platform (3) of the robotic device (4) opposite the support structure (15) that holds the first and second parts (5 and 6), wherein this support plate (31) also holds, if necessary, the actuating mechanism (26') of the control rod (26) that can move the locking pieces (22, 22') out of their locked position.

24. Device according to claim 23, **characterised in that** the actuators (24", 25") of the shafts (24', 25') come in the form of a double actuator that integrates a single motor (35) with high torque and with a low rotation speed, for example of the piezoelectric type, and an arrangement (36) for transmission gears that transform the rotational movement of the outlet of said motor into two synchronous rotational movements in opposite directions, provided with two shafts (24' and 25').

25. Device according to claims 12 and 23, **characterised in that** the support plate (31) also holds an actuator (17') for the threaded shaft (17), moving the cart (8), this, for example, in the form of a piezoelectric motor combined with a gear mechanism.

26. Device according to claim 2, **characterised in that** the jaws (7) that form the means for tightening the mandrel (5) are mounted guided in the tubular through passage (10") of the body (10), wherein said jaws (7) are equipped with drive means (39) that are stressed, directly or indirectly, by the surrounding manoeuvring ring (9), so as to be moved in translation along (Z or Z') during the rotation of this ring (9) by generating a simultaneous movement of mutual coming-together or moving-apart of said jaws (7) around the median axis of said passage (10") or an elongated body (2) mounted in said mandrel (5).

27. Device according to claim 26, **characterised in that** the tightening means (7) consist of two opposite and opposing jaws with mutually offset jaws (7") so as to interlock during their coming-together movement for the purpose of clamping, wherein the tubular passage (10") has two planar opposite faces (37) that form planar guide surfaces for said jaws (7), wherein the latter are also equipped with portions of lateral guide surfaces (38) that are suitable for sliding supported on said faces (37) and wherein each of said jaws (7) is guided in an additional manner during their mutual coming-together and moving-apart movements to carry out displacement in direction (Z or Z') simultaneously.

28. Device according to claim 27, **characterised in that** each of said jaws (7) is equipped with at least two drive and guide rods (39") that project laterally relative to its guide surface portions (38) and wherein each form a pair of opposite lugs or pins (39), wherein the latter are, on the one hand, mounted to slide in portions of grooves (40) that are made in the wall portions of the body (10) defining the planar opposite faces (37), coinciding two by two in the case of the two planar opposite faces (37) being superposed and located by coincident pairs in planes or sloped non-planar surfaces relative to the longitudinal direction (Z') of an elongated body (2) that is mounted in a tightened manner in the mandrel (5), and, on the other hand, at least for one pair of opposing pins or lugs (39) of each jaw (7), in direct or indirect drive connection with the manoeuvring ring (9) at their ends (39') that go through portions of grooves (40) through which they pass, in view of their movement in the longitudinal direction (Z') during a rotational movement of said manoeuvring ring (9).

29. Device according to any one of claims 27 or 28, **characterised in that** the ratio between the maximum inside opening of the mandrel (5), for maximum separation from the jaw (7), and the external diameter of the ring (9) is more than 0.2, preferably at least 0.3.

30. Device according to claim 28, **characterised in that** the body (10) has a circular-cylindrical exterior shape, adjusted in diameter to the inside diameter of the manoeuvring ring (9), the ends (39') of the pins or lugs (39) that exceed groove portions (40) at the outside face of the body (10) that is guided to slide into the grooves (41) that are made at the inside face of the manoeuvring ring (9) and formed by portions of curves each produced by the intersection between a regulated surface and the inside cylindrical surface of said ring (9), wherein the body (10) is locked in translation and is free in rotation in this surrounding ring (9).

31. Device according to claim 28, **characterised in that** the body (10) has a square or rectangular external shape and is mounted in the manoeuvring ring (9) with insertion of an intermediate piece (42) that has a circular-cylindrical external shape, adjusted in diameter to the inside diameter of the ring (9), and that has an inside shape that is complementary to the outside shape of the body (10), wherein this intermediate piece (42) is equipped, on the one hand, with grooves (43) coinciding by pairs, arranged by pairs in planes that are perpendicular to the median axis of the tubular passage (10") and that accommodate, with a guided sliding engagement, the ends (39') of the pins or lugs (39) that exceed portions of grooves (40) at the outside face of the body (10) and, on the other hand, at least two lugs (44) that project at its outside face and are guided to slide into grooves (41) that are essentially in the form of coil portions and are made at the inside face of the manoeuvring ring (9), wherein the body (10) is locked in translation and free in rotation in this surrounding ring (9).

32. Device according to any one of claims 26 to 31, **characterised in that** on its peripheral external face, the manoeuvring ring (9) has an inclined set of teeth (45) that is suitable for functional engagement with an endless screw (46) for the purpose of driving in rotation, the driving of said ring (9) beyond one degree of tightening determined from the elongated body (2) by the jaws (7), causing a rotation of the mandrel (5) and of the tightened body (2).

33. Device according to claims 3 and 3 or any one of claims 26 to 30 insofar as it relates to claims 2 and 3, **characterised in that** the mandrel (5) is secured, in a removable manner and with the capability of rotation, in a cart (8) that is mounted to slide in the translational direction (Z) in the elongated support structure (15) that is equipped with at least one suitable slide (47), wherein said structure (15) is equipped with an upper plate (15") for attachment to the platform (3) of the robotic device (4) and a lower plate (15') that comprises a guide opening or guide channel with play and with centring forming the second stationary part (6), and wherein a motorised assembly (48) for controlled movement of the cart (8) and a motorised assembly (49) for actuation of the manoeuvring ring (9) are combined with the cart (8)/support body (15) assembly by being mounted either on the platform (3) or on said support body (15).

34. Device according to claim 33, **characterised in that** the motorised assembly (48) consists of a mother screw (51) that is driven in rotation by a motor unit (52) that optionally is combined with a position coder and that drives in translation a wire nut (53), wherein said wire nut (53) is connected rigidly to a cart or a pair of intermediate guide carts (54) that slide on a rail (55) that is parallel to the translational direction (Z) and to the slide (47), wherein said cart or carts (54) is (are) also connected rigidly to a site (56') for removable coupling of a connecting bar (56) that is integral with the cart (8), wherein the points of attachment and connection of said wire nut (53) and of said coupling site (56') with the cart (54) or one of the carts (54) are connected to one another with insertion of a force sensor (54') that can measure the intensity of the mechanical actions in the direction (Z), and wherein said device comprises opposite ends-of-travel (57) that are mutually offset in the translational direction (Z) and that delimit the maximum travel of said intermediate cart or carts (54) and therefore of cart (8), wherein said motorised assembly (48), with the exception of the ends-of travel (57), is optionally mounted in a housing (60), formed by a single support with the platform (3) or accommodated, in an indexed way and with locking into position, in a receiving site (61) that is integral with the platform (3), by thus forming a removable autonomous module.

35. Device according to claim 33, **characterised in that** the motorised assembly (49) consists of a motor unit (58) that drives, via a flexible drive shaft (59), an endless screw (46) that is integrated in the cart (8) and is engaged in driving with the peripheral manoeuvring ring (9) of the mandrel (5) that is mounted in said cart (8), wherein said motor unit (58) is mounted in a housing that is formed by a single support with the platform (3) or accommodated in an indexed manner and with locking in position in a site for accommodating said platform (3).

36. Device according to any one of claims 1 to 35, **characterised in that** the elongated body (2) consists of a needle for implementing percutaneous medical procedures.

37. Device according to claims 13, 19, 21 and 33, **characterised in that** the platform (3) of the robotic device (4) has an open cutaway (3'), as well as optionally the upper plate (15") of the support structure (15), the separator (30) that holds the force sensors (29) and the support plate (31) of the actuators (17', 24', 25'), wherein the opening of this cutaway or these cutaways is oriented in the same direction as the openings (23, 23') of the mounting sites (16 and 19) of the first and second parts (5 and 6), and/or as a lateral release of the structure (15), so as to allow an installation and/or a lateral removal of said needle (2).

38. System for assisting with percutaneous medical procedures that are guided by medical imagery, comprising a robotic device for positioning and orienting a platform, a device for controlled insertion and retraction of the needle, and a device for piloting said devices based on preprogramed parameters and results of instantaneous measurements, such as measurements extracted from preoperative medical images, measurements of stresses in the axial direction of the needle and/or torque stresses at said needle, or else indications or measurements of position that are provided by the above-mentioned robotic device and/or insertion device, said system being **characterised in that** the device for inserting and retracting the needle consists of a device (1) according to any one of claims 1 to 37.

39. System according to claim 38, **characterised in that** the device (1) for inserting and extracting the needle (2) has a staged modular structure, with a first module (32) that contains the first and second parts (5 and 6) and the mechanical control means of the latter, wherein a second module (33) contains sensor means, and a third module (34) contains the means for actuating the above-mentioned mechanical control means, wherein the second and third modules (33 and 34) are packed in a sterile package, together with a portion that is adjacent to at least the robotic device (4) for positioning and orientation, and wherein the first module (32) is produced, partially or totally, as a module of the expendable or single-use type.

40. System according to claim 38, **characterised in that** the support body (15), if necessary with the cart (8) and optionally the hose (59), form(s) a module of the expendable or single-use type, wherein the additional portion of the system is packed in a sterile package.

## Patentansprüche

1. Vorrichtung zum Halten und zur gesteuerten Translationsbewegung eines länglichen Körpers in Form einer Stange vom Nadeltyp, und insbesondere einer Nadel für perkutane medizinische Verfahren, in seiner Längsrichtung, wobei die Vorrichtung zur Befestigung auf einer Halterung bestimmt ist, die in gesteuerter Weise hinsichtlich ihrer Position und Ausrichtung einstellbar ist, wie zum Beispiel einer Plattform oder einem Arm einer Robotervorrichtung zur Positionierung und Ausrichtung, wobei die Vorrichtung (1) im Wesentlichen einerseits eine erste Greifeinrichtung (5) zum Greifen des langgestreckten Körpers (2), die gegenüber der Halterung (3) in gesteuerter und/oder kontrollierter Weise in der Translationsrichtung (Z) mit einem vorgegebenen Maximalhub bewegbar ist, und andererseits eine zweite Einrichtung (6) umfasst, die gegenüber der Halterung (3) festgelegt ist und zur Führung des länglichen Körpers (2) bei seiner Bewegung durch die erste bewegliche Greifeinrichtung (5) und gegebenenfalls zum Halten des länglichen Körper (2) in seiner Position ausgebildet ist, wenn der letztere nicht in Eingriff mit dem ersten Einrichtung (5) steht, wobei die beiden Einrichtungen (5 und 6) in der Translationsrichtung (Z) ausgerichtet sind und die erste Einrichtung (5) in abwechselnder Weise zwischen einer proximalen Postion zu der zweiten Einrichtung (6) und einer von der letzteren entfernten Position bewegbar ist, wobei die bewegliche Greifeinrichtung (5) Einrichtungen (7) zum Einspannen durch Einklemmen und Blockieren des länglichen Körpers (2) aufweist, die in Drehung mit dem länglichen Körper (2) um die Translationsachse (Z) bewegt werden können, wobei die Vorrichtung (1) **dadurch gekennzeichnet ist, dass** die Greifeinrichtung (5) aus einem Spannfutter besteht, das den länglichen Körper vom Nadeltyp (2) in das Spannfutter durchquerender Weise aufnimmt, dass die Bewegungen des Einspannens und Entspannens des Spannfutters (5) durch eine Drehbetätigungsvorrichtung (9) gesteuert werden und dass die Klemmeinrichtungen (7) in Form von Klemmbacken mit der Möglichkeit eines geführten Gleitens in einem Körper (10) mit einem rohrförmigen Kanal (10") befestigt sind, der zur Aufnahme des länglichen Körpers (2) in einer diesen umgebenden und von diesem durchquerten Weise bestimmt ist, wobei die Bewegung der Klemmeinrichtungen (7) durch eine Drehbewegung der Betätigungsvorrichtung (9) erreicht wird.

2.  Vorrichtung nach Anspruch 1, **dadurch gekennzeichnet, dass** die Drehbetätigungsvorrichtung (9) die Form eines mit Zähnen oder Kerben versehenen Betätigungskranzes aufweist, der und mit dem Spannfutter (5) vereinigt ist, und dass die Bewegung der Klemmbacken (7) durch eine Drehbewegung des Betätigungskranzes (9) bewirkt wird, der den Körper (10) umgibt, wobei die Spannvorrichtung (5) in vorteilhafter Weise im offenen Zustand ein Ausmaß einer Öffnung aufweist, die ausreicht, um eine vollständige Freigabe des Körpers (2) mit lediglich einer seitlichen Halterung mit einer Hin- und Herbeweglichkeit zu ermöglichen.

3.  Vorrichtung nach Anspruch 1 oder 2, **dadurch gekennzeichnet, dass** das Spannfutter (5), das die bewegliche Greifeinrichtung bildet, auf oder in einem Schlitten (8) befestigt ist, der durch einen Motorantrieb in den zwei entgegengesetzten Richtungen der Translationsrichtung (Z) des länglichen Körpers (2) dadurch bewegbar ist, dass er in einer länglichen Halterungsstruktur (15) geführt ist, die zur starren Befestigung an der Plattform oder dem Arm (3) einer Robotervorrichtung (4) für die Positionierung und Ausrichtung ausgebildet ist, wobei die Halterungsstruktur in vorteilhafter Weise so geformt ist, dass sie einen seitlichen Zugang an einen in der Halterungsstruktur (15) in Eingriff stehenden Körper und gegebenenfalls eine Anordnung und Entfernung des Körpers (2) schräg seitlich oder radial, gegebenenfalls zusammen mit dem Spannfutter (5) ermöglicht.

4.  Vorrichtung nach Anspruch 1 oder 2, **dadurch gekennzeichnet, dass** das Spannfutter (5), das die bewegliche Greifeinrichtung bildet, direkt in einen länglichen Halterungskörper (15) mit der Möglichkeit eines geführten Gleitens in der Translationsrichtung (Z) des länglichen Körpers (2) und der Möglichkeit einer Drehung um die Achse (2) aufgenommen ist, wobei der Halterungskörper (15) eine Gleitvorrichtung für das bewegliche Spannfutter (5) bildet, dessen Bewegung vorzugsweise direkt aus einer manuellen Aktion eines Bedieners resultiert, wobei die Halterungsstruktur (15) vorzugsweise so geformt ist, dass sie einen seitlichen Zugang an den Körper (2), der in der Halterungsstruktur (15) in Eingriff steht, und gegebenenfalls eine Anordnung und Entfernung des Körpers (2) schräg seitlich oder radial ermöglicht.

5.  Vorrichtung nach einem der Ansprüche 3 und 4, **dadurch gekennzeichnet, dass** die zweite feste Einrichtung (6) im Bereich des Endes des Halterungskörpers (15) mit länglicher Struktur liegt, das distal bezüglich der Plattform oder dem Arm (3) angeordnet ist, die beziehungsweise der die Vorrichtung (1) trägt, und ein Körper (10') ist, der mit dem Halterungskörper (15) verbunden oder durch einen Teil des letzteren gebildet ist.

6.  Vorrichtung nach einem der Ansprüche 1 bis 5, **dadurch gekennzeichnet, dass** die zweite feste Einrichtung (6) aus einem an den länglichen Körper (2) angepassten Führungslager besteht, wobei die Reibung zwischen dem länglichen Körper und dem Lager zumindest ausreicht, um den länglichen Körper (2) gegen die Schwerkraft zu halten.

7.  Vorrichtung nach einem der Ansprüche 1 bis 5, **dadurch gekennzeichnet, dass** die zweite feste Einrichtung (6) aus einer Führungsöffnung oder einem Führungskanal mit Spiel für den länglichen Körper (2) besteht, die zur Begrenzung der radialen Hin- und Herbewegung oder Winkelneigung des länglichen Körpers (2) gegenüber der Translationsbewegung (Z) und zum Halten des länglichen Körpers (2) im Wesentlichen in einer koaxialen Anordnung zu der Richtung (Z) bestimmt ist, und zwar in Zusammenwirken mit dem Spannfutter (5), das die bewegliche Greif- und Antriebsvorrichtung bildet.

8.  Vorrichtung nach Anspruch 2, **dadurch gekennzeichnet, dass** die zweite feste Einrichtung (6) eine gesteuerte Greiffunktion für den länglichen Körper (2) verwirklicht und aus einem Spannfutter besteht, das den länglichen Körper (2) in das Spannfutter durchquerender Weise aufnimmt, wobei das zweite Spannfutter (6) eine Struktur, eine Betriebsart und Bestandteile aufweist, die identisch zu denen des ersten Spannfutters (5) sind, d.h. insbesondere einen Betätigungskranz (9') für Klemmbacken (7') und einen Körper (10') mit einem rohrförmigen Kanal (10").

9. Vorrichtung nach zumindest einem der Ansprüche 2 bis 8, **dadurch gekennzeichnet, dass** das oder jedes Spannfutter (5, 6) aus einem Körper (10, 10') mit einem diesen durchquerenden rohrförmigen Kanal (10") besteht, der seitlich offen ist, um den Durchgang von zumindest zwei, vorzugsweise drei Klemmbacken (7, 7') zu ermöglichen und mit Führungseinrichtungen (11, 11') für die Klemmbacken (7, 7') in einer Radialrichtung bezüglich des rohrförmigen Kanals des betreffenden Körpers (10, 10') versehen ist, dass die Klemmbacken (7, 7') gleichförmig um den Kanal (10") herum verteilt sind und zwischen einer Klemmstellung, in der sie sich teilweise in den entsprechenden Kanal (10") erstrecken, und einer Position beweglich sind, in der sie den Kanal (10") vollständig freigeben, und dass die Klemmbacken (7, 7') Segmente von Rippen/Nuten (12) aufweisen, die für einen Eingriff und für eine Bewegung in einer Nut/Rippe (13) mit einem spiralförmigen Teil ausgebildet sind, der in einer Seitenfläche des verzahnten Betätigungskranzes (9, 9') vorgesehen ist, der drehbeweglich auf einen Teil mit äußerer rohrförmiger Form (10"') des Körpers (10, 10') der betreffenden Spannvorrichtung (5, 6) befestigt sind, wobei eine Drehung des Kranzes (9 oder 9') ein gleichzeitiges und identisches radiales Gleiten der Klemmbacken (7 oder 7') antreibt.

10. Vorrichtung nach Anspruch 9, **dadurch gekennzeichnet, dass** das oder jedes Spannfutter (5, 6) drehbeweglich um die Längsachse (Z') des länglichen Körpers (2) befestigt ist, der dieses durchquert und gegebenenfalls mit diesem letzteren zusammengebaut ist, und dass jedes Spannfutter (5, 6) mit einem Rastmechanismus (14, 14') verbunden ist, der den Körper (10, 10') dieses betreffenden Spannfutters (5, 6) gegen eine Drehung in Richtung einer Entklemmung blockiert und eine fortschreitende Drehung des Spannfutters (5, 6) in der Klemmrichtung ermöglicht, wenn dieses eng um den länglichen Körper (2) gespannt ist, und ein zusätzliches Klemmmoment, das eine größere Stärke als ein vorgegebener Wert hat, der durch den betreffenden Rastmechanismus (14, 14') bestimmt ist, auf den entsprechenden Betätigungskranz (9, 9') ausgeübt wird.

11. Vorrichtung nach einem der Ansprüche 1 bis 8, **dadurch gekennzeichnet, dass** die erste bewegliche Einrichtung (5) und die zweite stationäre Einrichtung (6) vorzugsweise in lösbarer Weise auf einer Halterungsstruktur (15) befestigt sind, die zum starren Zusammenbau mit der Plattform oder dem Arm (3) einer Robotervorrichtung (4) zur Einstellung und Ausrichtung ausgebildet ist, wobei die Halterungsstruktur (15) in Form eines Chassis eine vorgegebene Erstreckung in der Richtung der Translationsbewegung (Z) des länglichen Körpers (2) aufweist, eine Befestigungsstelle (16) für die zweite feste Einrichtung (6) aufweist, die an einer Endstellung in der Richtung angeordnet ist, die einer positiven oder Eindringbewegung des länglichen Körpers (2) in der Translationsrichtung (Z) entspricht, und mit Einrichtungen (17, 18) für die Führung und die gesteuerte Bewegung der ersten beweglichen Einrichtung (5) versehen ist, die im Bereich einer entsprechenden angepassten Montagestelle (19) auf oder in einem Schlitten (8) befestigt ist, der mit den Einrichtungen (17, 18) zusammenwirkt.

12. Vorrichtung nach Anspruch 11, **dadurch gekennzeichnet, dass** die Einrichtungen zur Führung und gesteuerten Bewegung der ersten beweglichen Einrichtung (6) einerseits eine Gewindestange (17), die eine Leitspindel bildet, und andererseits zumindest eine Führungsschiene oder Führungsstange (18) umfassen, und dass der Schlitten (8) eine Gewindebohrung (8'), die eine Gewindemutter bildet, und zumindest ein Führungslager oder einen Gleitschuh (8") bildet, der gegebenenfalls durchgehend ist, und der in gleitender Weise mit der oder jeder Führungsschiene oder Führungsstange (18) zusammenwirkt.

13. Vorrichtung nach Anspruch 11 oder 12, **dadurch gekennzeichnet, dass** die ersten und zweiten Einrichtungen (5 und 6), die die Form von Spannfuttern aufweisen, in lösbarer Weise und der Möglichkeit einer Drehung an ihren jeweiligen im Wesentlichen U-förmigen Befestigungsstellen (16 und 19) aufgenommen sind, dass die Körper (10, 10') der Spannfutter (5, 6) mit allgemein kreiszylindrischer Form eine äußere Umfangsnut (20, 20') aufweisen, und dass die Befestigungsstellen (16, 19 jeweils eine Rippe (21, 21') aufweisen, die zum Zusammenwirken mit der Nut (20, 20') des Körpers (10, 10') des entsprechenden Spannfutters (5, 6) über einen Teil seiner Umfangserstreckung ausgebildet sind, um diese Körper (10, 10') zu halten, und dass die Befestigungsstellen (16, 19) seitlich offen sind, um das Anbringen und das Entfernen der Spannfutter (5, 6) zu ermöglichen, wobei die letzteren im montierten Zustand an ihren jeweiligen Befestigungsstellen (16, 19) durch bewegliche Blockierungsteile (22, 22') befestigt sind, die vorzugsweise in die Verriegelungsstellung der Spannfutter (5, 6) elastisch vorgespannt sind, wobei die Blockierungsteile (22, 22') mit den externen Nuten (20, 20') der Körper (10, 10') der betreffenden Spannfutter (5, 6) in der Verlängerung der Rippen (21, 21' in Eingriff stehen und die seitlichen Öffnungen (23, 23') der jeweiligen Befestigungsstellen (16, 19) verschließen.

14. Vorrichtung nach Anspruch 13, **dadurch gekennzeichnet, dass** die ersten und zweiten Einrichtungen (5, 6) in ihren jeweiligen Befestigungsstellen (19 und 16) gegen eine elastische Kraft befestigt sind wobei die letztere die Einrichtungen (5, 6) aus diesen Stellen (19 und 16) auswirft, wenn die Blockierungsteile (22, 22'), die vorzugsweise in gleichzeitiger Weise betätigt werden, die entsprechenden seitlichen Öffnungen (23 und 23') freigeben.

**15.** Vorrichtung nach einem der Ansprüche 3 und 5 bis 14, sofern diese auf den Anspruch 3 zurückbezogen sind, **dadurch gekennzeichnet, dass** der Schlitten (8), der die Greifeinrichtung (5) trägt, in lösbarer Weise mit der Vorrichtung (1) verbunden ist und insbesondere in eine extreme Gleitstellung in eine Höhenlage bewegbar ist, in der er sich von der Vorrichtung (1) trennt, wobei der Schlitten (8) in vorteilhafter Weise das Spannfutter (5) in lösbarer Weise und mit der Möglichkeit einer Drehung in einer ringförmigen Aufnahme mit angepasster Form aufnimmt, die eine durch einen lösbaren Deckel (62) verschließbare Einführöffnung aufweist.

**16.** Vorrichtung nach Anspruch 8 und einem der Ansprüche 9 bis 15 sofern diese auf den Anspruch 8 zurückbezogen sind, **dadurch gekennzeichnet, dass** der Antrieb des Betätigungskranzes (9, 9') jeder Einrichtung in Form eines Spannfutters (5, 6) über ein Ritzel (24, 25) verwirklicht ist, das mit dem Kranz (9, 9') in Eingriff steht und drehfest mit einer Stange oder Welle (24' 25') verbunden ist, die durch eine jeweilige entsprechende, gegebenenfalls gemeinsame Betätigungseinrichtung (24", 25") in Drehung angetrieben wird, dass das Ritzel (25), das mit dem Kranz (9') des stationären Spannfutters (6) kämmt, starr im Bereich des Endes einer entsprechenden Antriebswelle (25') mit dieser verbunden ist, und dass das Ritzel (24), das mit dem Kranz (9) des beweglichen Spannfutters (5) kämmt, drehfest bezüglich einer entsprechenden Antriebswelle (24'), jedoch für eine freie Translationsbewegung verbunden ist.

**17.** Vorrichtung nach Anspruch 13, **dadurch gekennzeichnet, dass** sie eine schwenkbare oder gleitende Betätigungswelle oder Betätigungsstange (26) aufweist, die antriebsmäßig mit den Blockierstangen (22, 22') zu deren Bewegung aus ihrer Verriegelungsstellung gegen elastische Vorspannkräfte verbunden ist, die beispielsweise durch Druckfedern (27, 27') geliefert werden, wobei diese Betätigungsstange (26) antriebsmäßig mit einem Betätigungsmechanismus (26') verbunden ist, insbesondere einen Sicherheitsmechanismus, der als Vorgabe eine Entriegelung hervorruft.

**18.** Vorrichtung nach den Ansprüchen 16 und 17, **dadurch gekennzeichnet, dass** die Wellen (24' und 25') sowie die Betätigungsstange (26) freie Enden auf der Seite der Betätigungseinrichtungen (24", 25") beziehungsweise des Mechanismus (26') aufweisen, die verjüngt oder angespitzt sind, und die insbesondere zum Durchbohren der Wand einer sterilen Umhüllung ausgebildet sind.

**19.** Vorrichtung nach einem der Ansprüche 16 bis 18, soweit diese auf dem Anspruch 11 zurückbezogen sind, **dadurch gekennzeichnet, dass** das die Halterungsstruktur (15) bildende Chassis eine untere Platte (15'), die die äußere Befestigungsstelle (16) für die zweite Einrichtung (6) verschließt, und eine obere Platte (15") für die Befestigung an der Plattform (3) einer Robotervorrichtung (4) zur Betätigung und Ausrichtung aufweist, wobei die obere Platte (15") mit Schnellbefestigungseinrichtungen (28), wie zum Beispiel Klinken versehen ist, die für den Eingriff mit der Plattform (3) oder einem an der letzteren befestigten Teil im montierten Zustand des Chassis (15) ausgebildet sind, und dass die Einrichtungen (17, 18) zur Führung und gesteuerten Bewegung sowie gegebenenfalls die Antriebswellen (24', 25') und die Betätigungsstange (26) sich zwischen den beiden Platten (15' und 15") erstrecken und zwischen diesen befestigt sind, deren gegenseitiger Abstand im Wesentlichen den Hub der ersten beweglichen Einrichtung (5) bestimmt.

**20.** Vorrichtung nach einem der Ansprüche 1 bis 19, **dadurch gekennzeichnet, dass** sie Kraftaufnehmer (29) umfasst, die die Intensität des auf den länglichen Körper (2) ausgeübten Druckes in der Translationsrichtung (Z) und gegebenenfalls des Drehmomentes oder bestimmter Teile zumindest des Drehmomentes messen, das auf den länglichen Körper (2) ausgeübt wird.

**21.** Vorrichtung nach Anspruch 20, sofern dieser auf dem Anspruch 19 zurückbezogen ist, **dadurch gekennzeichnet, dass** die Kraftaufnehmer (29) zwischen einer oberen Platte (15") der Halterungsstruktur (15) und einer Plattform (3) der Robotervorrichtung (4) und vorzugsweise auf einer Zwischenplatte (30) angeordnet sind, die zwischen der oberen Platte (15") und der Plattform (3) eingeschichtet ist.

**22.** Vorrichtung nach Anspruch 20 oder Anspruch 21, **dadurch gekennzeichnet, dass** die Kraftaufnehmer (29) aus einseitig in der Translationsrichtung (Z) gerichteten Kraftaufnehmern bestehen, von denen drei vorgesehen und in einer zur Richtung (Z) orthogonalen Ebene angeordnet und in symmetrischer Weise auf einem Kreis mit einem Radius (r) angeordnet sind, der auf der Längsachse (Z') des länglichen Körpers (2) zentriert ist, wobei diese Anordnung mit drei Aufnehmern (29) damit die Feststellung von zwei Komponenten des Reaktionsmomentes ermöglicht, das auf den länglichen Körper ausgeübt wird.

**23.** Vorrichtung nach den Ansprüchen 16 und 17, **dadurch gekennzeichnet, dass** die Betätigungseinrichtungen (24", 25"), die die Wellen (24', 25') antreiben, auf einer Tragplatte (31) gruppiert sind, die mit der Plattform (3) der Robotervorrichtung (4) entgegengesetzt zu der Halterungsstruktur (15) verbunden ist, die die ersten und zweiten Einrichtungen (5 und 6) trägt, wobei die Tragplatte (31) gegebenenfalls auch den Betätigungsmechanismus (26') für die Betätigungsstange (26) trägt, der zur Bewegung der Blockierungsteile (22, 22') aus ihrer Verriegelungsstellung ausgebildet ist.

24. Vorrichtung nach Anspruch 23, **dadurch gekennzeichnet, dass** die Betätigungseinrichtungen (24", 25") der Wellen (24', 25') die Form einer Doppelbetätigungseinrichtung aufweisen, die einen einzigen Motor (35) mit hohen Drehmoment und geringer Drehgeschwindigkeit, beispielsweise vom piezoelektrischen Typ, und eine Anordnung (36) von Getriebezahnrädern integriert, die die Drehbewegung am Ausgang des Motors in zwei synchrone Drehbewegungen mit entgegengesetzter Richtung umwandeln, die an die beiden Wellen (24') und (25') geliefert werden.

25. Vorrichtung nach den Ansprüchen 12 und 33, **dadurch gekennzeichnet, dass** die Tragplatte (31) außerdem eine Betätigungseinrichtung (17') für die Gewindestange (17) trägt, die den Schlitten (8) bewegt, beispielsweise in Form eines piezoelektrischen Motors, der mit einem Getriebemechanismus verbunden ist.

26. Vorrichtung nach Anspruch 2, **dadurch gekennzeichnet, dass** die Klemmbacken (7), die die Klemmeinrichtungen des Spannfutters (5) bilden, in dem rohrförmigen Durchgangskanal (10") des Körpers (10) geführt befestigt sind, wobei die Klemmbacken (7) mit Antriebseinrichtungen (39) versehen sind, die direkt oder indirekt durch den Umfangs-Betätigungskranz (9) angetrieben werden, derart, dass sie bei einer Drehung des Kranzes (9) in Translationsrichtung entlang (Z) oder (Z') angetrieben werden und eine gleichzeitige Bewegung der gegenseitigen Annäherung oder Entfernung der Klemmbacken (7) um die Mittelachse des Kanals (10") oder eines länglichen Körpers (2) erzeugen, der in dem Spannfutter (5) montiert ist.

27. Vorrichtung nach Anspruch 26, **dadurch gekennzeichnet, dass** die Klemmvorrichtungen (7) aus zwei entgegengesetzten Klemmbacken gegenüberliegend zu den Klemmbacken (7") bestehen, die derart gegeneinander versetzt sind, dass sie während ihrer Annäherung zur Einklemmung ineinander greifen, dass der rohrförmige Kanal (10") zwei gegenüberliegende ebene Flächen (37) aufweist, die ebene Führungsflächen für die Klemmbacken (7) bilden, wobei die letzteren in gleicher Weise mit Teilen von seitlichen Führungsflächen (38) versehen sind, die zum Gleiten in Anlage an den Flächen (37) ausgebildet sind, und dass jede der Klemmbacken (7) in zusätzlicher Weise während ihrer Bewegungen der gegenseitigen Annäherung oder Entfernung geführt sind, um gleichzeitig eine Bewegung in der Richtung (Z oder Z') zu bewirken.

28. Vorrichtung nach Anspruch 27, **dadurch gekennzeichnet, dass** jede der Klemmbacken (7) mit zumindest zwei Antriebs- und Führungsstangen (39") versehen ist, die seitlich gegenüber den Teilen der Führungsflächen (38) vorspringen und jeweils ein Paar von entgegengesetzten Zapfen oder Fingern (39) bilden, wobei die letzteren einerseits gleitend in Teilen von Nuten (40) montiert sind, die in den die gegenüberliegenden ebenen Flächen (37) bildenden Teilen der Wand des Körpers (10) vorgesehen sind, und die paarweise im Fall der Anordnung übereinander der beiden entgegengesetzten ebenen Flächen (37) zusammenfallen und die in zusammenfallenden Paaren in den nicht flachen Ebenen oder Oberflächen angeordnet sind und gegenüber der Längsrichtung (Z') eines länglichen Körpers (2) geneigt sind, der in eingeklemmter Weise in dem Spannfutter (5) befestigt ist, und andererseits zumindest ein Paar von entgegengesetzten Fingern oder Zapfen (39) jeder Klemmbacke (7) für einen direkten oder indirekten Antrieb mit dem Betätigungskranz (9) im Bereich ihrer Enden (39') bilden, die aus den Teilen der Nuten (40), die sie durchqueren, vorspringen, um eine Bewegung in der Längsrichtung (Z') bei einer Drehbewegung des Betätigungskranzes (9) hervorzurufen.

29. Vorrichtung nach einem der Ansprüche 27 und 28, **dadurch gekennzeichnet, dass** das Verhältnis zwischen der maximalen inneren Öffnung des Spannfutters (5) für eine maximale Entfernung der Klemmbacken (7) und dem Außendurchmesser des Kranzes (9) größer als 0,2 und vorzugsweise größer als 0,3 ist.

30. Vorrichtung nach Anspruch 28, **dadurch gekennzeichnet, dass** der Körper (10) eine äußere kreiszylindrische Form aufweist, die im Durchmesser an dem Innendurchmesser des Betätigungskranzes (9) angepasst ist, wobei die Enden (39') der Finger oder Zapfen (39), die aus den Teilen der Nut (40) im Bereich der Außenfläche des Körpers (10) vorspringen, in gleitenden geführten Eingriff mit Nuten (41) kommen, die im Bereich der Innenfläche des Betätigungskranzes (9) vorgesehen sind und durch Teile von Kurven gebildet sind, die jeweils am Schnittpunkt zwischen einer geregelten Oberfläche und der zylindrischen Innenoberfläche des Kranzes (9) entstehen, wobei der Körper (10) in Translationsrichtung blockiert und für eine freie Drehung in den umgebenden Kranz (9) ausgebildet ist.

31. Vorrichtung nach Anspruch 28, **dadurch gekennzeichnet, dass** der Körper (10) eine quadratische oder rechteckige Außenform aufweist und in dem Betätigungskranz (9) unter Einfügung eines Zwischenstückes (42) befestigt ist, das eine kreiszylindrische Außenform aufweist und in ihrem Durchmesser an den Innendurchmesser des Kranzes (9) angepasst ist und eine innere Form aufweist, die komplementär zu der äußeren Form des Körpers (10) ist, wobei das Zwischenstück (42) einerseits mit zueinander paarweise angeordneten Nuten (43) versehen ist, die in Paaren in Ebenen unter einem rechten Winkel zur Mittelachse des rohrförmigen Kanals (10'') angeordnet sind und mit einem geführten Gleiteingriff die Enden (39') von Fingern oder Zapfen (39) aufnehmen, die über die Teile der Nuten (40) im Bereich der Außenfläche des Körpers (10) vorspringen, und andererseits mit zumindest zwei Zapfen (44) versehen ist, die im Bereich ihrer Außenfläche vorspringen und im geführten Gleiteingriff mit Nuten (41) im Wesentlichen in Form von Teilen von Schraubenlinien kommen, die im Bereich der Innenfläche des Betätigungskranzes (9) ausgebildet sind, wobei der Körper (10) in einer Translationsrichtung festgelegt und frei drehbar in dem umgebenden Kranz (9) angeordnet ist.

32. Vorrichtung nach einem der Ansprüche 26 bis 31, **dadurch gekennzeichnet, dass** der Betätigungskranz (9) auf seiner äußeren Umfangsfläche eine geneigte Verzahnung (45) aufweist, die für einen funktionellen Eingriff mit einer Antriebsschnecke (46) derart ausgebildet ist, dass bei einem Drehantrieb ein Antrieb des Kranzes (9) über ein vorgegebenes Ausmaß der Einklemmung des länglichen Körpers (2) durch die Klemmbacken (7) eine Drehung des Spannfutters (5) und des eingeklemmten Körpers (2) hervorruft.

33. Vorrichtung nach den Ansprüchen 2 und 3 oder einen oder mehreren der Ansprüche 26 bis 30, soweit diese auf die Ansprüche 2 und 3 zurückbezogen sind, **dadurch gekennzeichnet, dass** das Spannfutter (5) in lösbarer Weise und mit der Möglichkeit einer Drehung in einem Schlitten (8) angeordnet ist, der in der Translationsrichtung (Z) gleitend in der länglichen Halterungsstruktur (15) befestigt ist, die mit zumindest einer angepassten Gleitführung (47) versehen ist, wobei die Struktur (15) mit einer oberen Platte (15'') für ihre Befestigung an der Plattform (3) der Robotervorrichtung (4) und einer unteren Platte (15') versehen ist, die eine Führungsöffnung oder einen Führungskanal mit Spiel und Zentrierung umfasst, der die zweite stationäre Einrichtung (6) bildet, und dass eine motorisierte Einheit (48) für eine kontrollierte Bewegung des Schlittens (8) und eine motorisierte Einheit (49) zur Betätigung des Betätigungskranzes (9) mit der Schlitten-(8)/Halterungskörper-(15)-Baugruppe verbunden und entweder auf der Plattform (3) oder auf dem Halterungskörper (15) befestigt sind.

34. Vorrichtung nach Anspruch 33, **dadurch gekennzeichnet, dass** die motorisierte Einheit (48) aus einer Leitspindel (51), die durch einen Schrittmotor (52) in Drehung angetrieben ist, der gegebenenfalls mit einem Positionskodierer verbunden ist, und eine Gewindemutter (53) in Translationsrichtung antreibt, wobei die Gewindemutter (53) mit einem Zwischenführungsschlitten oder einem Paar von Zwischenführungsschlitten (54) verbunden ist, die auf einer Schiene (55) parallel zur Translationsrichtung und der Gleitführung (47) gleiten, dass der oder die Schlitten (54) in gleicherweise starr mit einer Ankopplungsstelle (56') für eine lösbare Ankopplung einer Verbindungsstange (56) verbunden ist (sind), die mit dem Schlitten (8) verbunden ist, dass die Befestigungs- und Verbindungspunkte der Spindel (53) und der Ankopplungsstelle (56') mit dem oder einem der Schlitten (54) miteinander unter Einfügung eines Kraftaufnehmers (54') verbunden ist, der zur Messung der mechanischen Wirkung in der Richtung (Z) ausgebildet ist, und dass die Vorrichtung entgegengesetzte Hubende-Schalter (57) aufweist, die gegeneinander in der Translationsrichtung (Z) versetzt sind und den maximalen Hub des oder der Zwischenschlitten (54) und damit des Schlittens (8) begrenzen, wobei die motorisierte Einheit (48) mit Ausnahme der Hubende-Schalter (57) gegebenenfalls auf einem Rahmen (60) befestigt ist, der einstückig mit der Plattform (3) vereinigt ist oder in indexierter Weise und mit einer Blockierung der Position in einer Aufnahmestelle (61) angeordnet ist, die mit der Plattform (3) verbunden ist und somit ein autonomes lösbares Modul bildet.

35. Vorrichtung nach Anspruch 33, **dadurch gekennzeichnet, dass** der motorisierte Einheit (49) aus einem Motorblock (58) besteht, der über eine flexible Antriebswelle (59) eine Gewindeschnecke (46) antreibt, die mit dem Schlitten (8) vereinigt ist und in Antriebseingriff mit dem Umfangs-Betätigungskranz (9) des Spannfutters (5) steht, das in dem Schlitten (8) montiert ist, wobei der Motorblock in einem Rahmen befestigt ist, der einstückig mit der Plattform (3) vereinigt ist oder in indexierter Weise und mit einer Blockierung der Position an einer Aufnahmestelle der Plattform (3) aufgenommen wird.

36. Vorrichtung nach einem der Ansprüche 1 bis 35, **dadurch gekennzeichnet, dass** der längliche Körper (2) aus einer Nadel zur Durchführung von perkutanen medizinischen Verfahren besteht.

37. Vorrichtung nach den Ansprüchen 13, 19, 21 und 33, **dadurch gekennzeichnet, dass** die Plattform (3) der Robotervorrichtung (4) einen offenen Ausschnitt (3') ebenso wie gegebenenfalls die obere Platte (15'') der Halterungsstruktur (15) aufweist, wobei die Zwischenplatte (30) die Kraftaufnehmer (29) und die Halteplatte (31) der Betätigungseinrichtungen (17', 24', 25') trägt, wobei die Öffnung dieses oder dieser Ausschnitte in der gleichen Richtung wie die Öffnungen (23, 23') der Befestigungsstellen (16 und 19) der ersten und zweiten Einrichtungen (5 und 6) und/oder einer seitlichen Trennung der Struktur (15) derart ausgerichtet sind, dass eine seitliche Anbringung und/oder Entfernung der Nadel (2) möglich ist.

38. Unterstützungssystem für perkutane medizinische Verfahren, die durch medizinische Bilderzeugungstechniken geführt sind, mit einer Robotervorrichtung für die Positionierung und Ausrichtung einer Plattform, einer Vorrichtung zum kontrollierten Einführen und Zurückziehen einer Nadel, und einer Steuervorrichtung zur Steuerung dieser Vorrichtungen in Abhängigkeit von vorprogrammierten Parametern und den Ergebnissen von momentanen Messungen, wie zum Beispiel Messungen, die aus medizinischen voroperativen Bildern abgeleitet sind, von Messungen von Kräften in der axialen Richtung der Nadel und/oder von Momentenkräften im Bereich der Nadel oder von Anzeigen oder Messungen der Position, die von der Robotervorrichtung und/oder der genannten Einführungsvorrichtung geliefert werden, wobei das System **dadurch gekennzeichnet ist, dass** die Vorrichtung zur Einführung und zum Zurückziehen der Nadel aus einer Vorrichtung (1) nach einem der Ansprüche 1 bis 37 besteht.

39. System nach Anspruch 38, **dadurch gekennzeichnet, dass** die Vorrichtung (1) zum Einführen und Herausziehen der Nadel (2) eine mehrstufige modulare Struktur mit einem ersten Modul (32), das die ersten und zweiten Einrichtungen (5 und 6) und die Einrichtungen zur mechanischen Steuerung der letzteren einschließt, einem zweiten Modul (33), das die Kraftaufnehmer einschließt, und einem dritten Modul (34) aufweist, dass die Einrichtungen zur Betätigung der genannten mechanischen Steuerungseinrichtungen einschließt, wobei die zweiten und dritten Module (33 und 34) in einer sterilen Verpackung zusammen mit zumindest einem angrenzenden Teil einer Robotervorrichtung zur Positionierung und Ausrichtung aufbereitet sind und das erste Modul (32) teilweise oder vollständig als Modul von Einmal-Typ oder zur einmaligen Benutzung ausgebildet ist.

40. System nach Anspruch 38, **dadurch gekennzeichnet, dass** der Halterungskörper (15) gegebenenfalls mit dem Schlitten (8) und gegebenenfalls mit der flexiblen Einrichtung (59) ein Verbrauchsmodul oder ein Modul mit einmaliger Benutzung bilden, wobei der komplementäre Teil des Systems in einer sterilen Verpackung angeordnet ist.

Fig. 1

Fig. 4A

Fig. 4B

Fig. 2

Fig. 3

Fig. 5A

24",25"

35

17'

36

17

31

26

26'

34

Fig. 5B

Fig. 6

34

Fig. 7

Fig. 8A

Fig. 8B

Fig. 8

Fig. 9

Fig. 10

7, 7"  37  40

7, 7"

9

10

5  41

45

Fig. 11A

41

45

9

39, 39'  39"

38

7"  37  10"

38

39  39'

7  38  39'  39  7

7

40

Fig. 11B

40  5  10

Fig. 11

Fig. 12A

Fig. 12

Fig. 12B

3

5

2

15

6

10'

Fig. 13A

3

2

5

15"

15

Fig. 13

10', 15'

6

Fig. 13B

Fig. 14

Fig. 15

Fig. 16

62

5

46

8

Fig. 17

Fig. 18

**EP 1 871 265 B1**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 6726675 B
- FR 2005002357 W